(19) Europäisches Patentamt
European Patent Office
Office européen des brevets

(11) Publication number: 0 391 411 A2

# (12) EUROPEAN PATENT APPLICATION

(21) Application number: 90106510.2

(22) Date of filing: 05.04.90

(51) Int. Cl.[5]: C07H 19/073, C07H 19/173, A61K 31/70

(30) Priority: 06.04.89 US 334034
13.03.90 US 492450

(43) Date of publication of application:
10.10.90 Bulletin 90/41

(84) Designated Contracting States:
AT BE CH DE DK ES FR GB GR IT LI LU NL SE

(71) Applicant: BRISTOL-MYERS SQUIBB COMPANY
345 Park Avenue
New York, N.Y. 10154(US)

(72) Inventor: Sterzycki, Roman Z.
757 Opening Hill Road
Madison Connecticut 06443(US)
Inventor: Mansuri, Muzammil M.
1320 Deer Run Road
Cheshire Connecticut 06410(US)
Inventor: Martin, John C.
40 Brookside Palace
Cheshire Connecticut 06410(US)

(74) Representative: Kinzebach, Werner, Dr. et al
Patentanwälte Reitstötter, Kinzebach und Partner Sternwartstrasse 4 Postfach 86 06 49
D-8000 München 86(DE)

## (54) 3'-Substituted methyl nucleosides as antiviral agents.

(57) Certain 3′-substituted methyl nucleosides are disclosed which are useful in the treatment of viral infections, e.g., infections caused by herpes simplex virus types 1 and 2, and/or are useful as intermediates in the preparation of 3′-substituted methyl nucleosides having antiviral activity. In accordance with one aspect of the invention, there are provided novel 3′-substituted methyl nucleosides represented by Formulas I and II

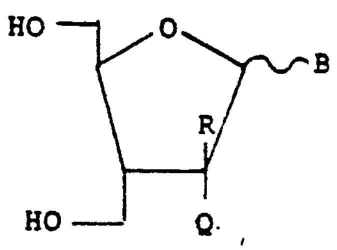

I

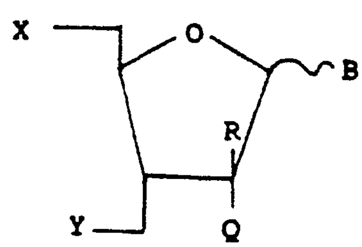

II

wherein:

B indicates that B can be either alpha or beta;

B is a member selected from the group consisting of the pyrimidine and purine nucleoside bases connected to the tetrahydrofuran ring through the $N^1$ heterocyclic nitrogen atom of the pyrimidines and the $N^9$ or $N^7$ heterocyclic nitrogen atom of the purines including the naturally occurring nucleoside bases and 5-fluoro, 5-bromo, 5-iodo, 5-chloro, 5-trifluoromethyl, 5-ethyl, 5-(2-bromovinyl)uracil; triazolecarboxamide; 2-aminopurine;

2,6-diaminopurine; 4-chloropyrimidine; pyrimidine; azapyrimidine; purine; 2,6-dichloropurine; 2-amino-6-chloropurine; and deazapurine;

R and Q are either both hydrogen or halogen, especially fluorine, or one is hydrogen and the other one is hydroxy or halogen, especially fluorine; in Formula I at least one of R and Q is halogen; and

EP 0 391 411 A2

X and Y can be the same or different and are selected from the group consisting of O-alkyl, O-aryl, O-acyl-, halogen, especially fluorine, azido, amino, acylamido, -SH, S-alkyl and S-aryl, or one of X or Y can be hydroxy, or X may be the group $R^1O$ and Y may be the group $R^2O$ wherein $R^1$ and $R^2$ are hydrogen or hydroxy-protecting groups.

Preferred O-alkyl and S-alkyl groups contain from one to four carbon atoms; preferred O-aryl and S-aryl groups contain from 6 to 10 carbon atoms; and preferred O-acyl groups contain from one to four carbon atoms.

# 3′-SUBSTITUTED METHYL NUCLEOSIDES AS ANTIVIRAL AGENTS

## Background of the Invention

This invention relates to 3′-substituted methyl nucleosides which are useful in the treatment of viral infections, e.g., infections caused by herpes simplex virus types 1 and 2 as well as cytomegaloviruses (CMV), varicella-zoster viruses (VZV) and retroviruses (HIV 1, HIV 2, MuLV, etc.).

Several 3′-hydroxymethyl nucleosides have been reported in the literature. Rosenthal et al, Canadian Journal of Chemistry, Vol. 47, pages 4477-4481 (1969) disclose a 3′-hydroxymethyl nucleoside having the formula:

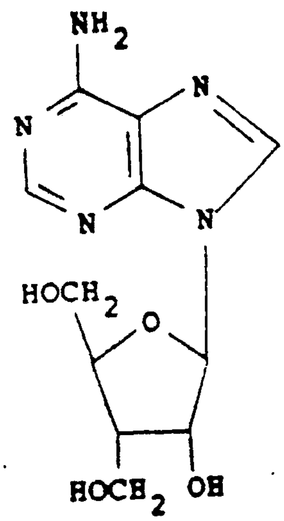

Shuto et al, Nucleosides and Nucleotides, Vol. 1, pages 263-273 (1982) disclose 3′-hydroxymethyl nucleosides having the formulas:

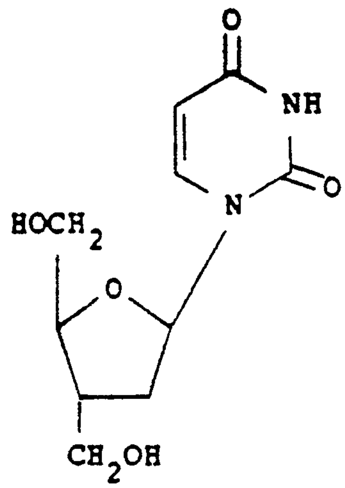

and

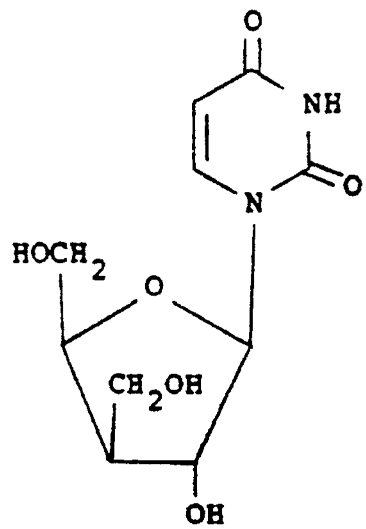

Similar information is disclosed in Japanese patent application Publication No. 57-146798, and the method disclosed is extended there to adenine intermediates, but preparation of the adenine end-products is not disclosed. Acton et al, Journal of Medicinal Chemistry, Vol. 22, No. 5, pages 518-525 (1979) disclose a 3′-hydroxymethyl nucleoside having the formula

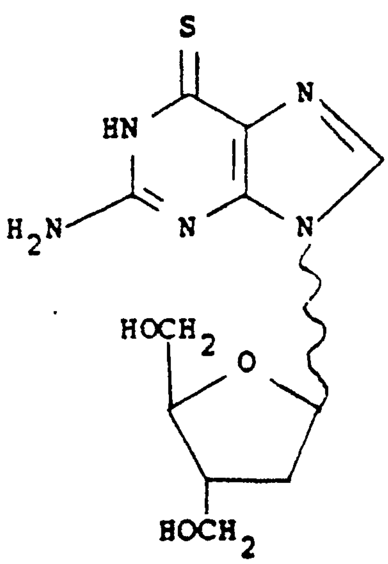

and that this compound has antitumor activity.

Oxetanacin A is a fermentation product having antibacterial and antiviral activity and the following structure (PharmaProjects, Vol. 10, May 1989, page M1051, PJB Publications Ltd., Richmond, Surrey, U.K.).

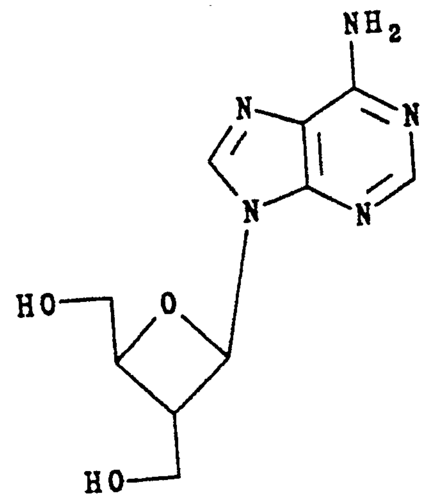

International Application No. PCT/SE88/00169, International Publication Number 88/08001, published October 20, 1988 discloses a compound of Formula A:

A

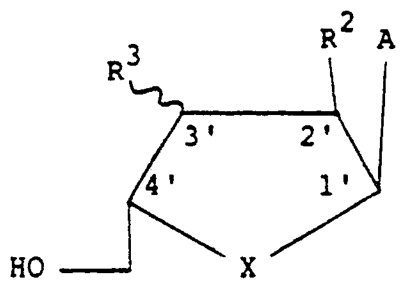

(a)

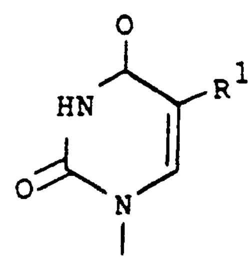

B

or

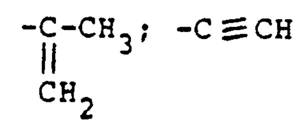

(b)

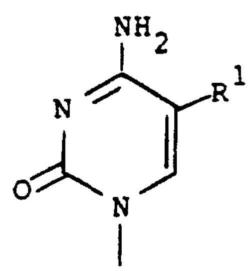

wherein the substituents A, X, $R^1$, $R^2$ and $R^3$ are defined as follows: A: formula (a) or formula (b); X: (a) O; (b) S; (c) $CH_2$; $R^1$: H; alkyl containing 1-3 carbon atoms; $-CH=CH_2$; $-CH=CH-CH_3$; $-CH_2-CH=CH_2$; formula (B); $-CH{\equiv}CH$; $R^2$: H; or $R^2$ constitutes together with $R^3$ a carbon-carbon bond; $R^3$: H; F; Cl; Br; I; $N_3$; CN; $C{\equiv}CH$; OH; $OCH_3$; $CH_2OH$; and when $R^3$ is F; Cl; Br; I; $N_3$; CN; $C{\equiv}CH$; OH; $OCH_3$ or $CH_2OH$ it may have either the cis-configuration or trans-configuration relative to the hydroxymethyl function at position 4′, or $R^3$ constitutes together with $R^2$ a carbon-carbon bond, and therapeutically acceptable salts thereof, for use in the treatment of HIV virus infections.

Brundidge et al (U.S. Patent 4,625,020) have disclosed a process for producing 1-halo-2-deoxy-2-fluoroarabinofuranosyl derivatives bearing protecting groups (Structure 1) from 1,3,5-tri-O-acyl-ribose. The 1-halo derivatives are intermediates in the synthesis of therapeutically active nucleoside analogues (Structure 2).

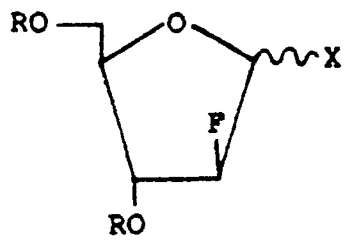

1

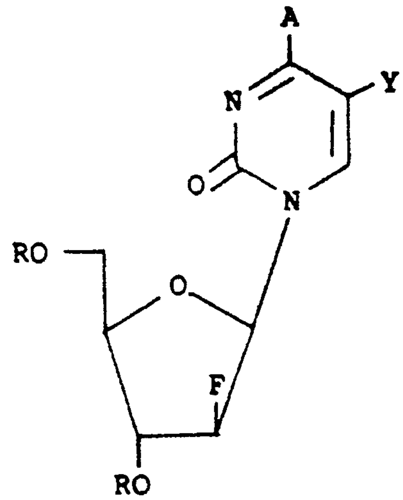

2

In Structures 1 and 2, R is an acyl group, X is halogen, especially bromine, A is OH or $NH_2$ and Y is halogen, alkyl or alkenyl. These compounds are of considerable importance due to their biological activity. Lopez et al (EP Patent Application No. 0,010,205) disclose that several 5-substituted 1-(2-deoxy-2-substituted-β-D-arabinofuranosyl)pyrimidine nucleosides (Structure 3) containing a 2′-fluoro substituent have biological activity. These compounds continue to be of considerable interest (Fox et al, "Antiviral Activities of 2′-Fluorinated Arabinosylpyrimidine Nucleosides" in "Fluorinated Carbohydrates" ed. N. F. Taylor, ACS Symposium Books No. 374, books 1988). Tuttle et al (EP 0,285,432) disclose that substituted 1-(2-deoxy-2-fluoro-β-D-arabinofuranosyl)purine nucleosides show good biological activity (Structure 4).

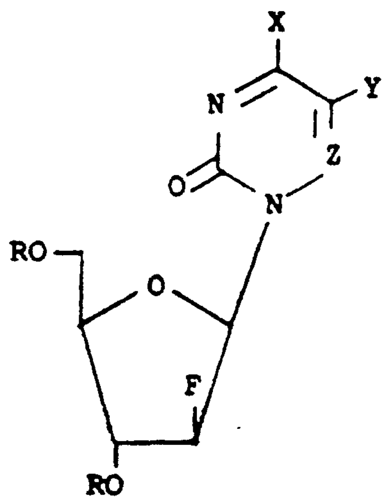

3

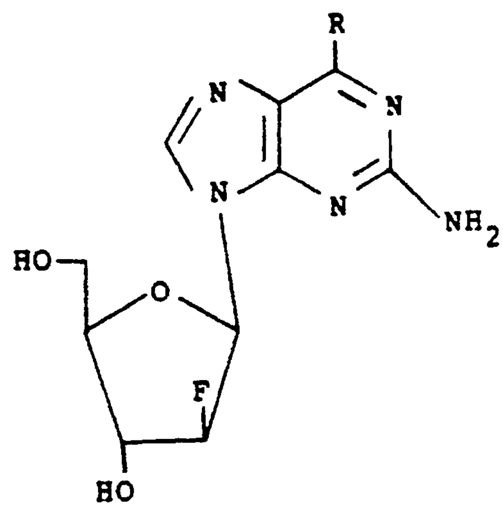

4

In Structure 3, each R may be hydrogen or acyl, X may be $NH_2$, Y may be halogen or alkyl, and Z may be methylene or nitrogen. In Structure 4, R may be hydrogen, hydroxy, etc.

Hertel (U.S. Patent No. 4,692,434) discloses that both pyrimidine and purine derivatives of 1-(2′-deoxy-2′,2′-difluoro-β-D-ribofuranosyl) nucleosides (Structures 5 and 6) show biological activity.

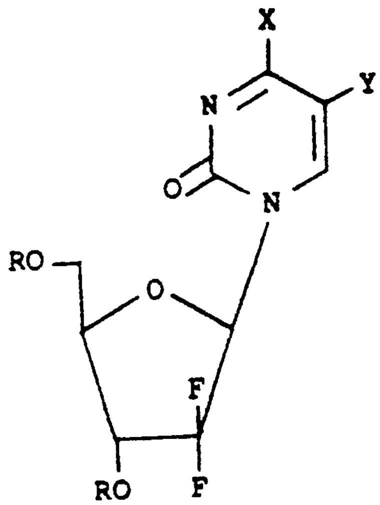

5

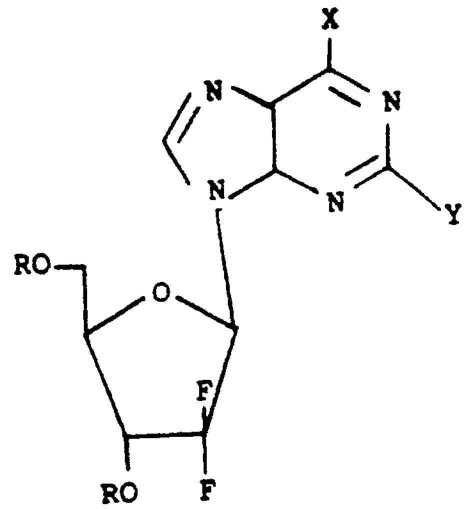

6

In Structures 5 and 6, each R may be hydrogen or acyl, and X and Y may each be hydrogen.

Matsuda et al (WO 88/07049) disclose novel 2-alkylidene pyrimidine nucleoside derivatives represented by Structure 7 having good antiviral effects.

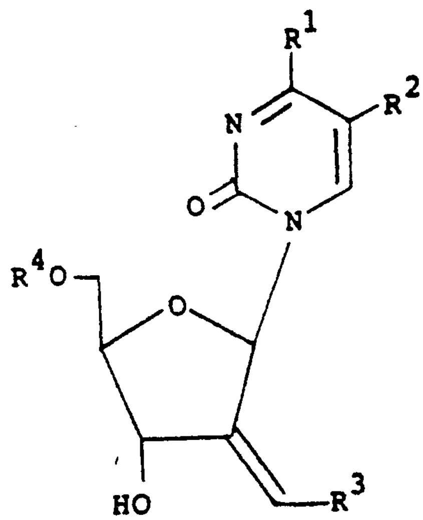

7

In Structure 7, $R^1$ is an amino group or hydroxy group, and $R^2$, $R^3$ and $R^4$ may all be hydrogen. A similar disclosure was made by Matsuda and others, J. Pharm. Pharmaceutical Sciences 76, No. 11, Nov. 1987.

## Summary of the Invention

It has now been found that certain 3′-substituted methyl nucleosides are useful in the treatment of viral infections, e.g., infections caused by herpes simplex virus types 1 and 2, and/or are useful as intermediates in the preparation of 3′-substituted methyl nucleosides having antiviral activity. Thus, in accordance with one aspect of this invention, there are provided novel 3′-substituted methyl nucleosides represented by Formulas I and II

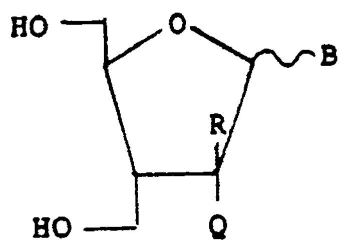

I

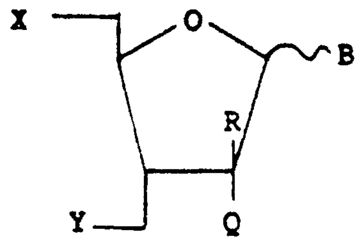

II

wherein:

~B indicates that B can be either alpha or beta;

B is a member selected from the group consisting of the pyrimidine and purine nucleoside bases connected to the tetrahydrofuran ring through the $N^1$ heterocyclic nitrogen atom of the pyrimidines and the $N^9$ or $N^7$ heterocyclic nitrogen atom of the purines including the naturally occurring nucleoside bases and 5-fluoro, 5-bromo, 5-iodo, 5-chloro, 5-trifluoromethyl, 5-ethyl, 5-(2-bromovinyl)uracil; triazolecarboxamide; 2-aminopurine; 2,6-diaminopurine; 4-chloropyrimidine; pyrimidine; azapyrimidine; purine; 2,6-dichloropurine; 2-amino-6-chloropurine; and deazapurine;

R and Q are either both hydrogen or halogen, especially fluorine, or one is hydrogen and the other one is hydroxy or halogen, especially fluorine; in Formula I at least one of R and Q is halogen; and

X and Y can be the same or different and are selected from the group consisting of O-alkyl, O-aryl, O-acyl-, halogen, especially fluorine, azido, amino, acylamido, -SH, S-alkyl and S-aryl, or one of X or Y can be hydroxy, or X may be the group $R^1O$ and Y may be the group $R^2O$ wherein $R^1$ and $R^2$ are hydrogen or hydroxy-protecting groups.

Preferred O-alkyl and S-alkyl groups contain from one to four carbon atoms; preferred O-aryl and S-aryl groups contain from 6 to 10 carbon atoms; and preferred O-acyl groups contain from one to four carbon atoms.

According to another aspect of this invention, there is administered to a host animal, including man, afflicted with a viral infection, e.g., an infection caused by herpes simplex virus types 1 or 2, a cytomegalovirus, a varicella-zoster virus or a retrovirus, a therapeutically effective amount of a 3′-substituted methyl nucleoside represented by Formulas I and II, wherein ~ B, B, R, Q, X and Y are the same as previously defined.

In accordance with still another aspect of this invention, there are provided novel intermediates useful in the preparation of the 3′-substituted methyl nucleosides represented by Formulas I and II.

## Detailed Description of the Invention

The synthesis of the 3′-substituted methyl nucleosides of Formulas I and II may be carried out by beginning with, among others, 1,2,5,6-diacetone-α-D-glucose or with 1,2-isopropylidene-α-D-xylofuranose as the starting materials. According to the preferred method, these materials are converted to the sugar derivative of Formula III

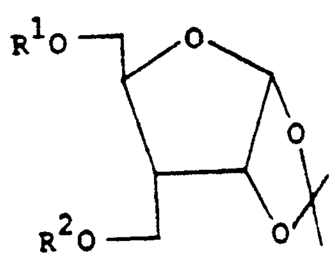

III

where $R^1$ and $R^2$ are conventional protecting groups, for example benzyl, benzoate or silyl groups, and can either be the same or different. For a range of the protecting groups $R^1$ and $R^2$ that can be used, see T. W. Greene "Protecting Groups in Organic Synthesis", 1981, John Wiley, New York, the disclosure of which is incorporated herein by reference. In the following discussion, $R^1$ and $R^2$ can either be the same or different and are as defined above for Formula III, and B is as defined above for Formulas I and II. The isopropylidene group can then be removed from the compound of Formula III and the compound acylated

to give the bisacylated product of Structure IV

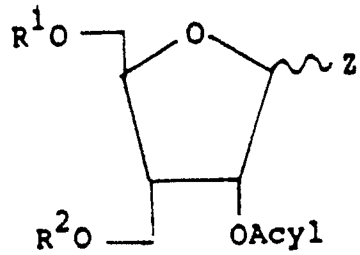

IV

wherein Z is an activated ester. These derivatives can be used directly or converted by well established procedures to the corresponding anomeric halides, especially bromide with TMSBr (which is trimethylsilyl bromide, see Gillard et al, Tetrahedron Lett., 1981, 22, 513), or see Brundidge et al, U.S. Patent 4,625,020) or different anomeric esters.

The carbohydrate intermediate IV can be treated with a non-activated or activated purine or pyrimidine base in the presence of one of a Bronsted acid and/or a Lewis acid to give a product of Formula V

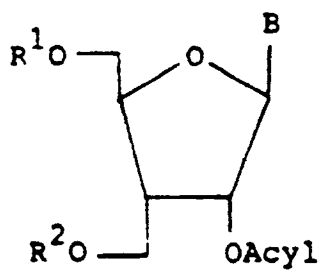

V

as mainly the β anomer. The base is activated by means of silylation or complexation with a heavy metal salt, preferably mercuric chloride, or by anion formation. The reaction uses either an aprotic solvent such as halogenated hydrocarbon (methylene chloride, chloroform, 1,2-dichloroethane, carbon tetrachloride, etc.), aromatic hydrocarbon (benzene, toluene, xylene etc.), or carboxylic acid derivative (ethyl acetate, acetonitrile etc.). One of the halogenated hydrocarbons is preferred at reaction temperatures between 0° to 140° C, usually from 30-80° C for from 0.5 hours to 10 days.

Deprotection under either acidic or basic or oxidative or reducing conditions or a combination thereof furnishes compounds of Formula VI

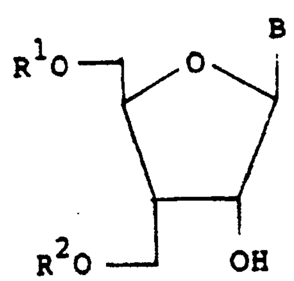

VI .

The exact choice of conditions will depend on the protecting groups used (see Greene, "Protecting Groups in Organic Synthesis", John Wiley, New York, 1981).

Compounds of Formula VI can also be deoxygenated at the 2′-position by a number of established procedures; for example, by removal of the 2′-protecting group under the appropriate reaction conditions and then formation of either the 2′-O-thiocarbonylimidazole ester (Prisbe et al, Syn. Comm., 1985, 15, 401) or the phenylthionocarbonate (Robins et al, J. Am. Chem. Soc., 1981, 103, 932) at the 2′-position. Reaction of the thiocarbonyl derivative with $Bu_3SnH$ gives the desired products of Formula VII.

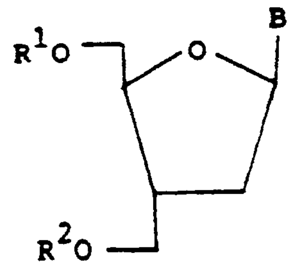

VII

The deoxygenation sequence uses either an aromatic hydrocarbon solvent (benzene, toluene, xylene etc.) or an ethereal solvent (THF, dioxane etc.) or dipolar aprotic solvent (DMF, which is dimethyl formamide; HMPA, which is hexamethylphosphoric triamide, etc.). The reaction should be conducted at 20-140 °C, preferably 60-120 °C, for 1-36 hours. Deprotection of compounds of Formula VII can be achieved under acidic or basic or oxidizing or reducing conditions or a mixture thereof. The exact choice of conditions will depend on the protecting groups used. In those cases wherein the protecting groups at positions 5′ and 6′ are the same, then direct removal furnishes compounds of Formula VIII.

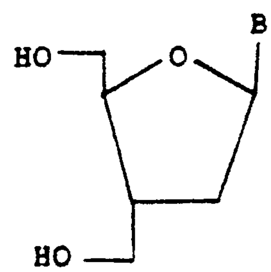

VIII

If $R^1$ and $R^2$ are different, the groups can be removed sequentially at the 5′- and 6′- positions (or vice versa). $R^2$ can be removed under the conditions required to liberate a free OH at the 6′-position and leave the 5′- position protected and furnish a compound of Formula IX

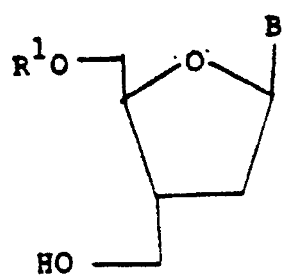

IX .

Alternatively, $R^1$ can be removed preferentially to liberate the 5′-OH while leaving the 6′-position protected to afford an intermediate of Formula X

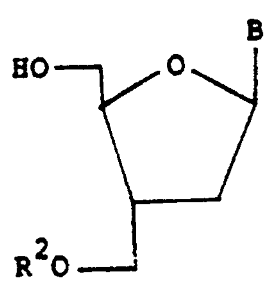

X

Nucleoside analogues IX and X can both be converted to derivatives of Formula VIII by removal of the

final protecting group. The deprotection conditions will depend upon the exact protecting groups used (see Greene, "Protecting Groups in Organic Synthesis, supra).

Compounds of Formula IX can be activated at the 6′-OH with a suitable leaving group, usually an alkyl or arylsulfonate, by reaction with alkylsulfonyl or arylsulfonyl chloride in the presence of an organic base (pyridine, triethylamine etc.) in halogenated hydrocarbon solvents (methylene chloride, 1,2-dichloroethane etc.) at -20-80° C for 1-24 hours. The activated intermediate thus produced can then be deprotected at the 5′-position under appropriate conditions to remove the $R^1$-protecting group (Greene, "Protecting Groups in Organic Synthesis", supra). Displacement of the activating group on the 6′-position with a suitable nucleophile (azide, bromide, fluoride, etc.) will furnish compounds of Formula II (X = OH, Y = nucleophile). The displacement reactions can be conducted in halogenated hydrocarbons (1,2-dichloroethane etc.), aromatic hydrocarbons (toluene, xylene), carboxylic acid derivatives (acetonitrile) or dipolar aprotic solvents (dimethylformamide) in the range of 20-150° C, preferably 60-100° C for 1-24 hours.

In an identical manner, derivatives of Formula X can be activated at the 5′-position by a suitable activating group and then the protecting group on the 6′-position removed. Displacement with the desired nucleophile in this instance at the 5′-position will again furnish derivatives of Formula II (X = nucleophile, Y = OH).

In an alternative approach to the β-anomeric nucleosides of Formulas I and II, the intermediates of Formula III can be converted to the novel dimer Intermediate XI.

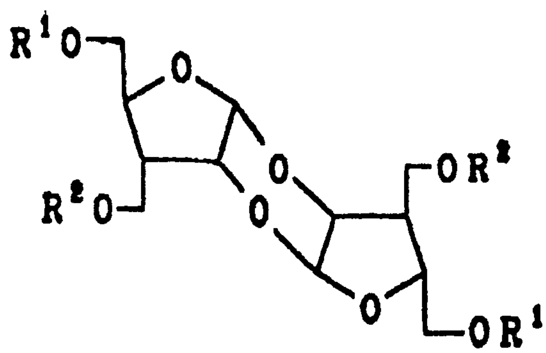

XI

Dimer formation is conducted in a halogenated hydrocarbon solvent (methylene chloride, chloroform etc.) in the presence of ferric chloride and silica. The preferred reaction conditions require a reaction temperature of 40-120° for 2 hours to 4 days.

Direct ring opening of the dimer of Formula XI with a suitably activated nucleoside base (vide supra) gives compounds of Formula VI. These are identical to the compounds prepared by the earlier method. Manipulations of the structures produced by this method in exactly the same manner as before leads to compounds of Formulas I and II.

The nucleoside analogues of Formula VI where B is a pyrimidine base can be activated at the 2′-OH position with an activating group, usually an alkyl or arylsulfonate or a thiocarbonylimidazole group. Under the preferred reaction conditions, the reaction should be carried out by reacting the derivatives of Formula VI with the corresponding alkyl or arylsulfonyl chloride or thiodiimidazole in halogenated hydrocarbon solvents (methylene chloride, 1,2-dichloroethane, etc.) or ethereal solvents (THF, dioxane) or dipolar aprotic solvents (DMF, HMPA etc.) at -20-120° C to give compounds of Formula XII.

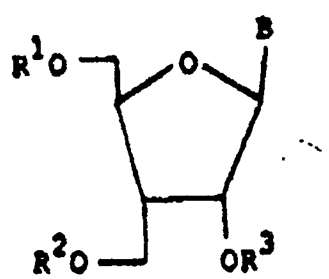

XII

wherein $R^3$ is a leaving group such as alkylsulfonate, arylsulfonate, trifluoroalkylsulfonate or other well

established leaving groups. The reactions with alkyl or arylsulfonates require the presence of an organic base. Subsequent treatment of the activated intermediates of Formula XII under either basic or thermal conditions or a combination in ethereal solvents (THF, dioxane, etc.) or aromatic hydrocarbon solvents (benzene, toluene, etc.) or amide solvents (acetonitrile, DMF, etc.) at 20-140° C for 1 to 36 hours affords the derivatives of Formula XIII.

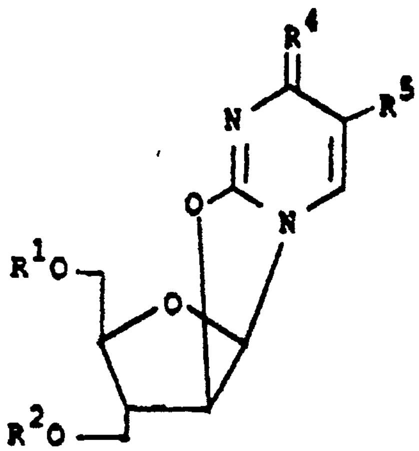

XIII

wherein $R^4$ is O or NH, and $R^5$ is halo, alkyl or alkenyl.

Treatment of derivatives of Formula XIII under aqueous basic conditions afforded nucleosides of Formula XIV

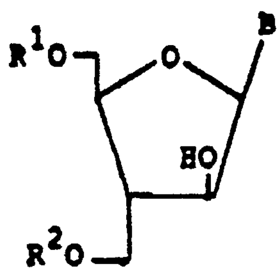

XIV

Removal of the protecting groups either at the same time ($R^1$ = $R^2$) or sequentially ($R^1 \neq R^2$) under appropriate conditions (see Greene "Protecting Groups in Organic Synthesis", supra) gave the nucleosides of Structure XV

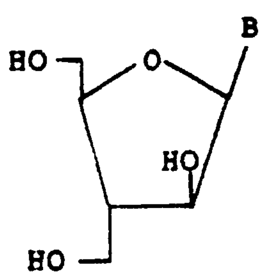

XV

where B is a pyrimidine base.

Derivatives of Formula III on treatment with aliphatic or aromatic alcohols (C1-C4) in the presence of either a Lewis and/or Bronsted acid are readily transformed to derivatives of Formula XVI.

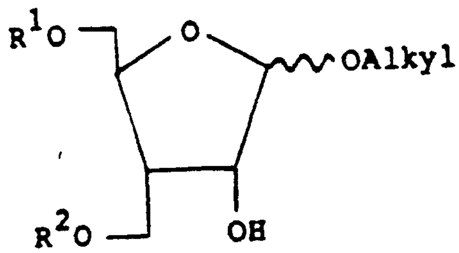

XVI

Subsequent deoxygenation at the 2-position by one of the methods described above gave the derivatives of Formula XVII.

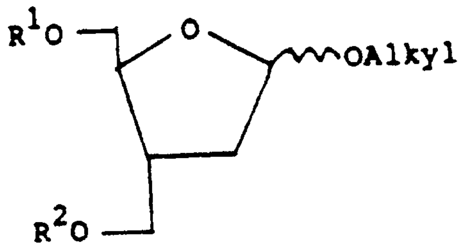

XVII

These can be converted by well established procedures to the corresponding anomeric esters or halides of Formula XVIII

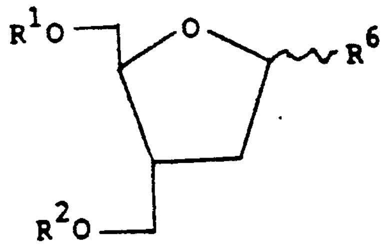

XVIII

wherein $R^6$ is a halide or ester. (See Brundidge et al, U.S. Patent 4,625,020 or by using TMSBr, see Gillard et al, supra).

Coupling reaction of these carbohydrates of Formula XVII or XVIII with a suitably activated base (for method of activation see above) gives an anomeric mixture of compounds of Formula XIX.

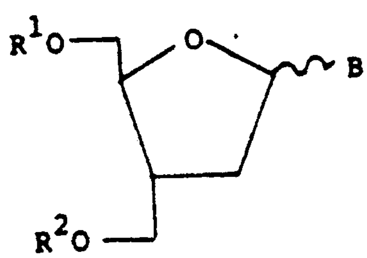

XIX

This anomeric mixture can be separated by chromatographic and crystallization techniques well known to those in the field to which this invention relates. Removal of the protecting groups of either the pure α or pure β anomer gives analogues of Formula I. The protecting groups can be removed together ($R^1 = R^2$) or separately ($R^1 \neq R^2$). The exact conditions will depend upon the protecting groups used.

In an alternative approach to the anomeric mixture XIX, derivatives of Formula VII which are β anomers can be converted to an anomeric mixture of Formula XIX. The β-anomers can be transglycosylated chemically under acidic conditions (Eckstein et al, J. Org. Chem., 1978, 43, 3044). Under these conditions, a nucleoside of known anomeric configuration is converted to an anomeric mixture of a nucleoside carrying a different nucleic base moiety. These reactions normally convert pyrimidine nucleosides to purine nucleosides. We have also used these reactions to prepare anomeric mixtures from pure anomers using the

same base moiety. In the preferred case, the β-anomer of Formula VII is reacted with a silylated purine or pyrimidine base in the presence of a Lewis acid catalyst in either a dipolar aprotic solvent (DMF, HMPA) or acid derived solvent such as acetonitrile or an aromatic hydrocarbon solvent at 20-140° C for 1-48 hours to give the anomeric mixture of products XIX after work up. Derivatives of Formula XIX can be purified as described before and converted to analogues of Formula I.

The 2′-fluoro containing nucleosides should be prepared as follows. Compounds of Formula XXXI may be prepared by the method of Fox et al (Carb. Res. 1975, 42, 233). These derivatives are convertible to their 3′-hydroxy derivatives XXXII

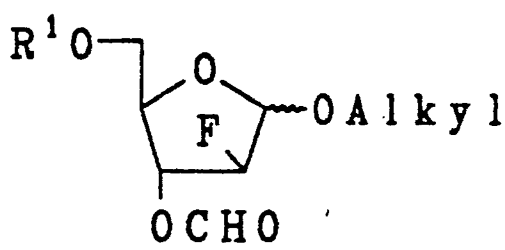

XXXI

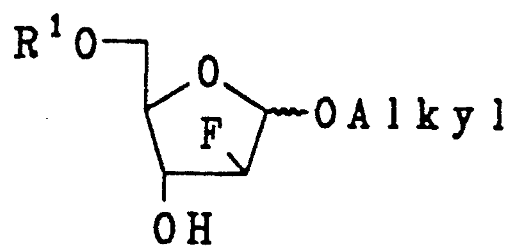

XXXII

under mild basic conditions ($NaHCO_3$/MeOH). Derivative XXXII can be oxidized to the ketone under conventional Jones oxidation conditions to furnish derivative XXXIII.

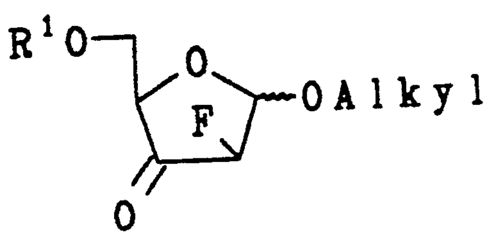

XXXIII

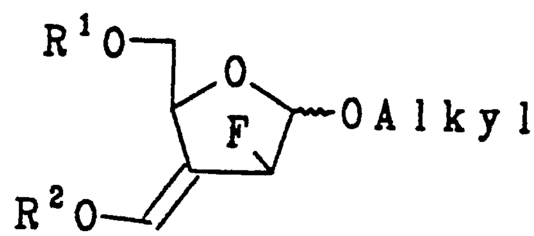

XXXIV

Homologation of XXXIII with the Wittig or modified Wittig reagent [cf: Tetrahedron Letter, 1982, 23 4293] will furnish derivatives of Compound XXXIV.

Hydrogenation of XXXIV using any of several catalysts (see Compendium of Organic Synthetic Methods) will furnish a mixture of products at the 3′-position, namely Compound XXXV. These can be separated by one skilled in the art to furnish the isomers shown as Formulas XX and XXXVI.

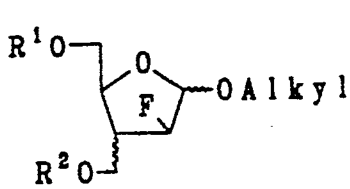

XXXV

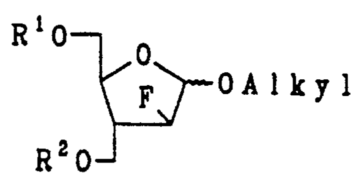

XX

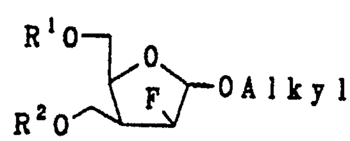

XXXVI

a: α anomer

b: β anomer

Alternatively, removal of the 5′-protecting group of XXXIV would give XXXVII, which can then be reduced

with hydrogen to provide XXXVIII. Protection of the free alcohol would furnish XX.

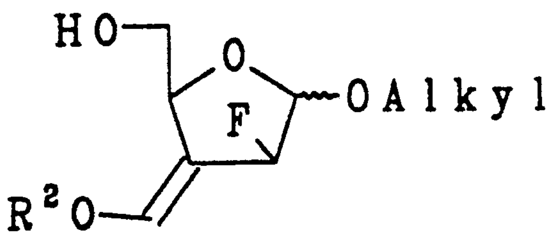

XXXVII

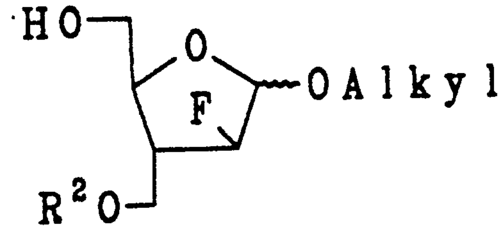

XXXVIII.

The derivatives of Formula XX can be converted by well established procedures to the corresponding anomeric esters or halides of Formula XXI

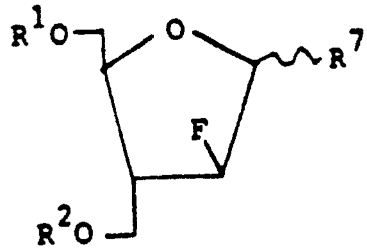

XXI

wherein $R^7$ is bromide or activated ester (TMSBr or see Brundidge et al, U.S. Patent 4,625,020). The fluorinated sugar of Formula XX or XXI can be reacted with a non-activated or activated purine or pyrimidine base (the manner of activation is described above) to furnish a mixture of structure of Formulas XXIIa and XXIIb.

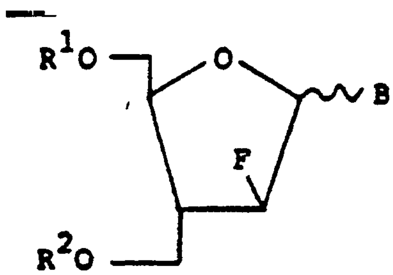

XXII

XXII a: α anomer
b: β anomer

The compounds XXIIa and XXIIb wherein $R^1$ and $R^2$ are protecting groups can then be deprotected to give the pure anomers of Formula XXIIIa and XXIIIb.

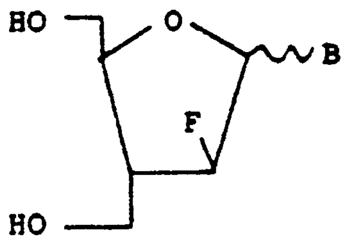

XXIII

XXIII a: α anomer
b: β anomer

If $R^1$ and $R^2$ are the same, then the groups can be removed together. If $R^1$ and $R^2$ are different, then the groups must be removed sequentially. The exact conditions for the deprotection will depend upon the protecting groups used.

In an alternative procedure, either $R^1$ or $R^2$ can be removed preferentially to leave either the 5′-position free (Formula XXIV)

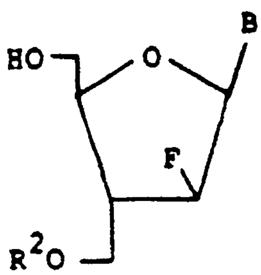

XXIV

or the 6′-position free (Formula XXV)

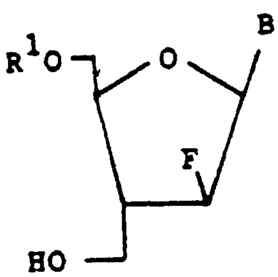

XXV

by suitable choice of reaction conditions (see Greene, "Protecting Groups in Organic Synthesis", supra). Compounds XXIV and XXV can be converted to derivatives of Formula II in an analogous sequence to that used to convert derivatives of Formula IX and X to Formula II.

The ribo 2′-fluoro derivatives of general Structure XXVI

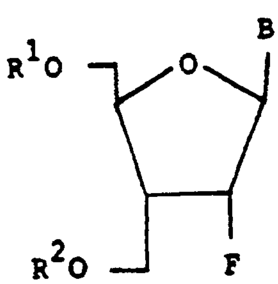

XXVI

are best derived from compounds of Formula XIV. Reaction of XIV directly with diethylaminsulfur trifluoride

(DAST) (Herdewijn et al, J. Med. Chem., 1987, 30, 2131) gives the 2′-ribo fluoro derivatives of Formula XXVI. Alternatively, the compounds of Formula XIV can be activated at the 2′-OH position as either an alkyl or arylsulfonate or a trifluoroalkylsulfonate by reaction with the appropriate alkyl or aryl sulfonylchloride or trifluoroalkylsulfonyl anhydride at temperatures ranging from -20-80°C, preferably in the 0-30° range. Subsequent displacement with a fluoride ion source such as tris(dimethylamino)sulfonium difluorotrimethyl silicate (TASF, Szarek et al Can. J. Chem., 1987, 65, 233) will give the compounds of Formula XXVI. Alternatively, Formula XXVI may be prepared directly from XIII. Treatment of XIII with KF in dimethylformamide in the presence of a crown ether (Mengel et al, Angew. Chemie, Int. Edn., 1978 17, 525) will give Formula XXVI. Deprotection of the compounds of Formula XXVI under the appropriate conditions will give structures of Formula XXVII.

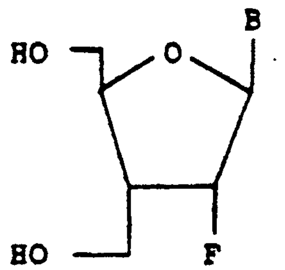

XXVII

The exact conditions will depend upon the nature of $R^1$ and $R^2$. As before, the protecting groups can be removed together if they are the same ($R^1 = R^2$) or sequentially if they are different ($R^1 \neq R^2$).

The protected derivatives of XXVI β-anomer can be converted to the anomeric mixture, in the same method as described to convert derivatives VII to XIX under acidic or other conditions, to give compounds of Formula XXVIII.

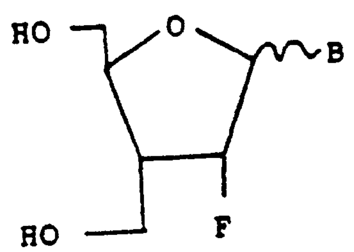

XXVIII

The 2′-olefinic derivatives of Formula XXIX

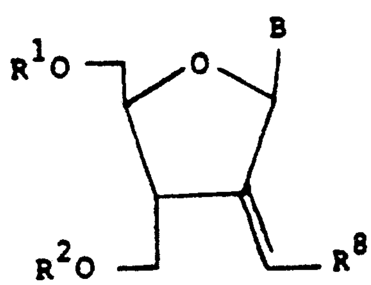

XXIX

wherein $R^8$ is hydrogen or alkyl, may be prepared from compounds of Formula VI. Oxidation of the derivatives of Formula VI gives the 2′-keto derivatives.

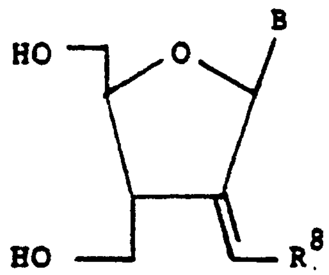

XXX

Treatment of 2′-keto derivatives with the appropriate triphenyl phosphonium salt (Wittig reagent) will give the protected nucleosides of Formula XXIX (Matsuda et al, J. Pharm. Sci., 1987, 76, S224).

Deprotection of derivatives XXIX under the appropriate conditions to remove the protecting groups will give compounds of Formula XXX.

The 3′-substituted methyl nucleosides of Formulas I and II have pronounced antiviral activity and may be used in the treatment of various human and animal diseases caused by viruses, such as herpes simplex virus types 1 and 2. Especially preferred compounds for the treatment of viral infections are those of Formula I wherein R is hydrogen and Q is hydrogen or hydroxy. The compounds of Formulas I and II are administered in any physiologically acceptable method, e.g., parenterally, in a therapeutically effective amount. Determination of optimum dosages is within the skill of the art.

The following examples illustrate the preparation of the compounds of Formulas I and II. In these examples, THF is tetrahydrofuran, TMCS is trimethylsilyl chloride, AIBN is azobisisobutyronitrile, DMSO is dimethylsulfoxide, EtOH is ethyl alcohol, iPrOH is isopropylalcohol and HMDS is hexamethyl disilazane.

## Example 1

1,2-Di-O-acetyl-5-O-benzyl-3-deoxy-3-[(benzyloxy)methyl]-β-D-ribofuranose.

1,2-O-Isopropylidene-3-deoxy-3-[(benzyloxy)methyl]-5-O-benzyl-α-D-ribofuranose, prepared by the process of Acton et al, supra, (52 mg; 0.135 mmol) was heated at 80-90 °C in 80% acetic acid (3 mL) for 6 h. Volatiles were removed in vacuo and the residue was dissolved in dry pyridine (2.5 mL). Acetic anhydride (1 mL) was added and the mixture was kept at room temperature for 14 h. After removal of volatiles in vacuo, the residue was filtered through silica (20% to 25% ethyl acetate in hexane) to give the title compound (45 mg; 77.8% yield) containing about 30% of the α-D-isomer. PMR(360 MHz; $CDCl_3$): 7.24-7.34(m, 10, aromatic), 6.38(d, J = 4.4 Hz, 0.3, H-1 α-anomer), 6.05(s, 0.7, H-1 β-anomer), 5.34(dd, $J_1$ = 8.6 Hz, $J_2$ = 4.4 Hz, 0.3, H-2 α-anomer), 5.28(d, J = 4.9 Hz), 4.54(bs, 2, benzylic $CH_2$) 4.41(m, 2, benzylic $CH_2$), 4.34(m, 0.3, H-4 α-anomer) 4.19 (m, 0.7 H-4 β-anomer) 3.68-3.50(m, 4, H-5 + H-6), 2.79-2,75(m, 1, H-3), 2.01(s, 0.9, $OCOCH_3$, α-anomer), 2.00(s, 0.9, $OCOCH_3$ α-anomer), 1.99(s, 2.1, $OCOCH_3$ β-anomer), 1.91(s, 2.1, $OCOCH_3$, β-anomer).

## Example 2

1,2,5-Tri-O-acetyl-3-deoxy3-[(acetoxy)methyl]-β-D-ribofuranose.

α-D-1,2,O-isopropylidene-3-deoxy-3-C-hydroxymethyl ribofuranose, prepared by the process of Acton et al, supra, (4.0 g; 19.59 mmol) was acetylated with excess of pyridine and acetic anhydride overnight. Evaporation yielded α-D-1,2-O-isopropylidene-3-deoxy-3-[(acetoxy)methyl]-5-O-acetyl-ribofuranose (5.6 g) which was used without purification for subsequent steps.

PMR(300 MHz; $CDCl_3$): 5.81(d, J = 3.6 Hz, 1, H-1), 4.67(bt, J = 3.9 Hz, 1, H-2), 4.36-4.29(m, 2, $CH_2OCOCH_3$), 4.15-4.06(m, 2, $CH_2OCOCH_3$), 2.25(m, 1, H-3), 2.04(s, 3, $OCOCH_3$), 2.02(s, 3, $OCOCH_3$), 1.46(s, 3, acetonide $CH_3$), 1.28(s, 3, acetonide $CH_3$).

This reaction product was dissolved in 60% acetic acid (30 mL) and heated at 100 °C for 5 h. Volatiles

were removed in vacuo and the residue was acetylated as described above. After evaporation, the crude product was filtered through silica (40% to 50% ethyl acetate in hexane) to give the title product (2.7 g; 43.3% yield overall) containing 20% of the α-D-isomer. PMR(200 MHz; $CDCl_3$): 6.38(d, 0.2, H-1 α-anomer), 6.08(s, 0.8, H-1 β-anomer), 5.27(d, J = 4.8 Hz, 0.8, H-2 β-anomer), 4.33-4.07(m, 5, 2 x $CH_2OCOCH_3$ + H-4), 2.69(m, 1, H-3) , 2.08 + 2.07 + 2.05 + 2.04(s, 4 x $OCOCH_3$).

## Example 3

Methyl 5-O-benzyl-2,3-dideoxy-3-[(benzyloxy)methyl]-β-D-erythro-pentofuranoside.

Methyl 5-O-benzyl-3-deoxy-3-[(benzyloxy)methyl-β-D-ribofuranoside, prepared by the process of Acton et al, supra, (4.5 g; 12.56 mmole) was dissolved in dry THF (100 mL) and treated with thiocarbonyldiimidazole (8.91 g; 50 mmole). The reaction mixture was heated under reflux for 14 h. The solvent was evaporated and the residue was purified on silica (10% to 18% of ethyl acetate in dichloromethane) to give pure 2-O-thiocarbonylimidazole derivative (5.1 g; 87.6% yield). A solution of this material (4.9 g; 10.57 mmole) in dry toluene (300 mL) was added dropwise to a refluxing solution of the tributyltin hydride (6.46 mL; 24.00 mmol) under argon in dry toluene (500 mL). The solution was heated to reflux for 40 h, the volatiles were removed in vacuo and the residue was purified by chromatography on silica (hexane to 25% of ethyl acetate in hexane). Yield: 2.3 g (63.55%) of the title compound with spectral properties as described by Acton et al, supra.

## Example 4

1-(2,3-Dideoxy-3-C-hydroxymethyl-β-D-erythro-pentofuranosyl)thymine and 1-(2,3-dideoxy-3-C-hydroxymethyl-α-D-erythro-pentofuranosyl)thymine.

Dry thymine (1.26 g; 10 mmol) was silylated and the crude product dissolved in dry 1,2-dichloroethane (50 mL) together with methyl 5-O-benzyl-2,3-dideoxy-3-[(benzyloxy)methyl-β-D-erythro-pentofuranoside (779 mg, 2.275 mmol). To this solution, trimethylsilyl triflate (1.0 mL; 4.87 mmole) was added and the reaction mixture was stirred at room temperature for 2 h under argon. 1,2-Dichloroethane (50 mL) was added and the whole was washed with a cold saturated bicarbonate solution. Drying and evaporation gave 1.1 g of the crude material which was carefully chromatographed on silica (25% to 60% of ethyl acetate in hexane). The β-D and α-D compounds (170 mg) were dissolved in ethanol (6 mL) and cyclohexene (20 mL), 20% $Pd(OH)_2/C$ (70 mg) was added and the mixture heated under reflux for 45 minutes. Filtration through Celite (washed with ethanol) and evaporation gave a crude mixture of free nucleosides, which was purified on a $C_{18}$ μBondapak column (19 x 150 mm) using 10% to 20% methanol in water. 1-(2,3-Dideoxy-3-C-hydroxymethyl-α-D-erythropentofuranosyl)thymine was eluted first and subsequently crystallized from methanol - diethyl ether (25 mg; 4.5% yield): m.p. 78-80 °C. PMR(360 MHz; $D_2O$): 7.56(bs, 1, H-6) , 6.09(t, J = 7.1 Hz, 1, H-1′), 4.21(m, 1, H-4′), 3.78(dd, $J_1$ = 2.7 Hz, $J_2$ = 12.4 Hz, 1, A of ABX of H-5′), 3.65(d, J = 3.2 Hz, 2, H-6′), 3.615(dd, $J_1$ = 5.6 Hz, $J_2$ = 12.5 Hz, 1, B of ABX, H-5′), 2.59(m, 1, H-3′), 2.43(m, 1, H-2′), 1.95(m, 1, H-2′), 1.86(s, 1, $CH_3$). CMR(90.56 MHz; $D_2O$) : 1 68.24(C-4), 153.21(C-2) , 138.79(C-6), 112.62(C-5), 87.89(C-1′), 84.78(C-4′), 64.06 + 63.13(C-5′ + C-6′), 42.73(C-3′), 36.13(C-2′), 12.95($CH_3$) . MS-EI(m/e) : 2.55($M^+$-H), 224($M^+$ - $CH_3OH$), 206($M^+$ - OH - $CH_3OH$), 130($M^+$ -thymine).

1-(2,3-Dideoxy-3-C-hydroxymethyl-β-D-erythro-pentofuranosyl)thymine was eluted second and crystallized from methanol - diethyl ether (50 mg; 9% yield): m.p. 122-124 °C PMR(360 MHz, $D_2O$) : 7.71(bs, 1, H-6), 6.09(dd, $J_1$ = 4.3 Hz, $J_2$ = 7.0, 1, H-1′), 3.93(m, 1, H-4′), 3.87(dd, $J_1$ = 12.6 Hz, $J_2$ = 2.8 Hz, 1, A of ABX, H-5′), 3.715(dd, $J_1$ = 12.6 Hz, $J_2$ = 5.0 Hz, 1, B of ABX, H-5′), 3.64 (d, J = 5.9 Hz, 1, H-6′), 2.46 (m, 1, H-3′), 2.25(m, 2, H-2′), 1.85(bs, 3, $CH_3$). CMR(90.56 MHz; $D_2O$): 168.04(C-4), 153.18(C-2), 139.02 (C-6), 115.41(C-5), 86.72 + 85.07(C-1′ + C-4′), 63.48 + 63.35(C-5′ + C-6′), 41.45(C-3′), 35.78(C-2′), 12.96-(thymine $CH_3$). MS-EI(m/e): 255($M^+$ - H), 224($M^+$ -$CH_3OH$), 206($M^+$ - OH -$CH_3OH$), 130($M^+$ - thymine). M.S. - high resolution (FAB-glycerol) for $C_{11}H_{17}N_2O_5$: Calc.: 257-1137. Found: 257-1142.

## Example 5

1-[2,5-Di-O-acetyl-3-deoxy-3-[(acetoxy)methyl]-β-D-ribofuranosyl)uracil.

Dry uracil (100 mg; 0.89 mmol) and β-D-1,2,5-tri-O-acetyl-3-deoxy-3-[acetoxymethyl]-β-D-ribofuranose containing 20% of the α-D isomer (250 mg; 0.786 mmole) were successively treated (stirring, argon) with dry acetonitrile (10 mL), TMCS (0.102 mL); 0.80 mmol), HMDS (0.168 mL; 0.80 mmol) and tin tetrachloride (0.142 mL; 1.0 mmol) solution in acetonitrile (5 mL). The reaction mixture was stirred at room temperature for 2 h and diluted with dichloromethane (40 mL). Washing with saturated bicarbonate, drying ($Na_2SO_4$), and evaporation gave the crude product (225 mg), which was subsequently purified on silica (elution with 30% ethyl acetate in dichloromethane to 60% ethyl acetate and 4% ethanol in 36% dichloromethane). Yield of the title product: 124 mg (41%). PMR(300 MHz; $CDCl_3$): 7.42(d, J = 8.4 Hz, 1, H-6), 5.71(dd, $J_1$ = 8.1 Hz, J = 2.1 Hz, 1, H-5), 5.66(d, J = 2.4 Hz, 1, H-1′), 5.48 (dd, $J_1$ = 6.6 Hz, $J_2$ = 2.1 Hz, 1, H-2′), 4.10 - 4.03(m, 5, H-4′ + H-5′ + H-6′), 2.75(m, 1, H-3′), 2.10(s, 3, $OCOCH_3$), 2.095(s, 3, $OCOCH_3$), 2.025(s, 3, $OCOCH_3$).

## Example 6

1-(3-Deoxy-3-C-hydroxymethyl-β-D-ribofuranosyl)uracil.

1-[2,5-Di-O-acetyl-3-deoxy-3-[(acetoxy)methyl]-β-D-ribofuranosyl]uracil (230 mg; 0.598 mmol) was dissolved in methanol (15 mL) saturated at room temperature with ammonia gas. The reaction mixture was stirred at room temperature for 16 h, volatiles were removed in vacuo, and the residue recrystallized from ethanol to give the title compound (86.2 mg, 55.82% yield). M.p. 201-203 °C. PMR(300 MHz; $D_2O$): 7.98(d, J = 8.1 Hz, 1, H-6), 5.82(d, J = 8.1 Hz, H-5), 5.78(s, 1, H-1′), 4.50(d, J = 5.2 Hz, 1, H-2′), 4.18(m, 1, H-4′), 3.97 (dd, $J_1$ = 2.6 Hz, $J_2$ = 13 Hz, 1, H-5′), 3.87 -3.67(m, 3, 2 x H-6′ + H-5′), 2.33(m, 1, H-3′) . For $C_{10}K_{14}N_2O_6$ x 1/3 $H_2O$ Calcd.: 45.72% C, 5.57% H, 10.67% N. Found: 45.88% C, 5.47% H, 10.62% N.

## Example 7

1-[2,5-Di-O-acetyl-3-deoxy-3-[(acetoxy)methyl]-β-D-ribofuranosyl] cytosine.

1-[2,5-Di-O-acetyl-3-deoxy-3-[(acetoxy)methyl]-β-D-ribofuranosyl]uracil (250 mg); 0.65 mmol) dissolved in dry pyridine (5 mL) was treated with 4-chlorophenyl phosphorodichloridate (326 µL; 2.0 mmol) and sublimed 1,2,4-triazole (277 mg; 4.0 mmol). This mixture was stirred for 16 h under argon (room temperature). Dioxane (3 mL) and concentrated ammonia (0.7 mL) were added and stirring was continued for 48 h. Volatiles were removed in vacuo, the residue was taken up in ethyl acetate and pyridinium hydrochloride was filtered off. The crude product was purified on silica (5% methanol in dichloromethane to pure methanol) to give the title compound (119 mg; 47.7% yield). PMR(300 MHz; $CDCl_3$) : 7.53(d, J = 7.5 Hz, 1, H-6), 5.77 (d, J = 7.5 Hz, 1, H-5), 5.67(bs, 1, H-1′), 5.59(bd, 1, H-2′), 4.39 - 4.22(m, 5, H-4′ + H-5′ + H-6′), 2.70(m, 1, H-3′). CMR(75.46 MHZ; $CDCl_3$): 166.79(C-4), 156.1(C-2), 141.73(C-6), 95.27(C-5), 93.02-(C-1′), 81.11(C-4′), 77.5(C-2′), 64.29 + 60.12 (C-5′ + C-6′), 40.98(C-5′), 20.97($CH_3CO$).

## Example 8

1-(3-Deoxy-3-C-hydroxymethyl-β-D-ribofuranosyl)cytosine.

1-[2,5-Di-O-acetyl-3-deoxy-3-[(acetoxy)methyl]-β-D-ribofuranosyl]cytosine (50 mg; 0.13 mmol) was dissolved in methanol (5 mL) saturated with ammonia gas. The reaction mixture was stirred at room temperature for 16 h, volatiles removed in vacuo, and the residue crystallized from ethyl methanol - ethyl acetate to give the title compound (18 mg; 53.8% yield). M.p. 223-226 °C. PMR(300 MHZ; DMSO $d_6$): 8.045(d, J = 7.4 Hz, 1, H-6), 7.12 + 7.00 (2 x bs, 2, $NH_2$), 5.65(d, J = 7.4 Hz, 1, H-5), 5.60(s, 1, H-1′), 5.51(d, J = 4.8 Hz, 1, H-2′), 5.06(t, J = 5.1Hz, 1, OH), 4.07(t, J = 5.1 Hz, 1, OH), 3.92(m, 1, H-4′), 3.81 - 3.38 (m, 4, H-5′ + H-6′), 2.08(m, 1, H-3′).
For $C_{10}H_{15}H_3O_5$ calcd.: 46.69% C, 5.88% H, 16.33% N. Found: 46.52% C, 5.91% H, 16.23% N.

## Example 9

1-[2-O-Acetyl-5-O-benzyl-3-deoxy-3-[(benzyloxy)methyl]-β-D-ribofuranosyl]uracil.

To a solution of 1,2-di-O-acetyl-5-O-benzyl-3-deoxy-3-[(benzyloxy)methyl]-β-D-ribofuranose (9.1 g; 21.24 mmol) in dry acetonitrile (300 mL) was added dry uracil (2.576 g; 23.0 mmol), HMDS (5.23 mL; 25 mmol), TMCS (3.37 mL; 27 mmol) and trifluoromethanesulfonic acid (2.36 mL; 27 mmol). This mixture was stirred for 3.5 h at room temperature under argon and then for 40 minutes at 60 °C. Excess solvent was removed under vacuum, chloroform (200 mL) was added and the solution was washed with saturated bicarbonate and brine. Drying and evaporation afforded a crude product (11.85 g), which was purified on silica (50% of ethyl acetate in hexane to 5% ethanol in 1:1 ethyl acetate in hexane) to give the pure product (8.05 g; 78.87% yield). PMR(300 MHz; $CDCl_3$): 7.89(d, J = 8.1 Hz, 1, H-6), 7.36 - 7.23 (m, 10, aromatic), 5.94(d, J = 2.7 Hz, 1, H-1′), 5.9 (dd, $J_1$ = 2.5 Hz, $J_2$ = 6.1 Hz, 1, H-2′), 5.30 (dd, $J_1$ = 8.1 Hz, $J_2$ = 2.1 Hz, 1, H-5), 4.52(narrow AB, 2, benzyl $CH_2$), 4.45 (narrow AB, 2, benzyl $CH_2$), 4.22(dm, J = 8.4 Hz, 1, H-4′), 3.93(dd, $J_1$ = 2.1 Hz, $J_2$ = 10.8 Hz, 1, H-5′), 3.65 - 3.53(m, 3, 2 x H-6′ + H-5′), 2.81(m, 1, H-3′), 1.995(s, 3, $OCOCH_3$).

## Example 10

1-[5-O-Benzyl-3-deoxy-3-[(benzyloxy)methyl]-β-D-ribofuranosyl] uracil.

1-[2-O-acetyl-5-O-benzyl-3-deoxy-3-[(benzyloxy)methyl]-β-D-ribofuranosyl]uracil (8.0 g; 16.65 mmol) was dissolved in methanol (175 mL) saturated with ammonia gas and the solution was stirred at room temperature for 16 h. Evaporation yielded the title compound (7.32 g; quant). PMR(300 MHZ; $CDCl_3$): 8.17-(d, J = 8.1 Hz, 1, H-6), 7.35 -7.23(m, 10, aromatic), 5.72 (s, 1, H-1′), 5.21(d, J = 8.1 Hz, 1, H-5), 4.51 (narrow AB, 2, benzyl $CH_2$), 4.49 (AB, 2, benzyl $CH_2$), 4.32(m, 2, H-2′ + H-4′), 4.0 (dd, $J_1$ = 2.1 Hz, $J_2$ = 11.1 Hz, 1, H-5′), 3.80 - 3.58(m, 3, H-5′ + 2 x H-6′), 2.57(m, 1, H-3′).

## Example 11

1-[5-O-Benzyl-2,3-dideoxy-3-[(benzyloxy)methyl]-β-D-erythro-pentofuranosyl]uracil.

1-[5-O-benzyl-3-deoxy-3-[(benzyloxy)methyl]-β-D-ribofuranosyl]uracil (3.9 g; 8.894 mmol) dissolved in dry 1,2-dichloroethane (80 mL) was treated with thiocarbonyldiimidazole (1.786 g; 10 mmol). The reaction mixture was heated under reflux under argon for 10 minutes. PMR indicated complete conversion of the starting material to the 1-[2-O-imidazolothiocarbonyl-5-O-benzyl-3-deoxy-3-[(benzyloxy)methyl]-β-D-ribofuranosyl]uracil, which was used without purification. A solution of this material in dry THF (45 mL) was added dropwise to a refluxing solution of the bistributyltin oxide (20 mL), polymethylhydrosiloxane (20 mL) and AIBN in dry toluene (180 mL) under argon. Heating was continued for a further 1.5 h, the reaction mixture was allowed to cool, and the volatiles were removed in vacuo. Purification on silica (50% ethyl

acetate in hexane to 10% ethanol in 1:1 ethyl acetate in hexane) gave the title compound (2.95 g; 78.5% yield). PMR(300 MHz, $CDCl_3$): 7.99(d, J = 7.8 Hz, 1, H-6), 7.32 - 7.19(m, 10, aromatic), 6.065(bdd, $J_1$ = 3.3 Hz, $J_2$ = 5.4 Hz, 1, H-1′), 5.29(d, J = 7.8 Hz, 1, H-5), 4.53(bs, 2, benzyl $CH_2$), 4.48(bs, 2, benzyl $CH_2$), 4.04(bd, J = 6.9 Hz, 1, H-4′), 3.90 (d, J = 10.2 Hz, 1, H-5′), 3.64(d, J = 11.1 Hz, 1, H-5′), 3.46(m, 2, H-6′), 2.68(m, 1, H-3′), 2.30 - 2.00(m, 2, H-2′).

## Example 12

1-(2,3-Dideoxy-3-C-hydroxymethyl-β-D-erythro-pentofuranosyl)uracil.

1-[5-O-Benzyl-2,3-dideoxy-3-[(benzyloxy)methyl-β-D-erythro-pentofuranosyl]uracil (450 mg; 1.065 mmol) dissolved in ethanol (7 mL) and cyclohexene (20 mL) was heated under reflux with $Pd(OH)_2/C$ (700 mg) for 16 h. The catalyst was filtered off (Celite) and the product was purified on silica and crystallized from ethanol to give the title compound (139 mg; 57.4% yield). PMR(300 MHz; DMSO $d_6$): 7.98(d, J = 8.1 Hz, 1, H-6), 5.95 (dd, $J_1$ = 4.3 Hz, $J_2$ = 6.8 Hz, 1, H-1′), 5.58(dd, $J_1$ = 1.9 Hz, $J_2$ = 8.0 Hz, 1, H-5), 5.05 (t, J = 5.1 Hz, 1, OH), 4.79 (t, J = 5.1 Hz, 1, OH), 3.77 (m, 1, H-4′), 3.69 - 3.50(m, 2, H-5′), 3.43(m, 2, H-6′), 2.32(m, 1, H-3′), 2.16(m, 1, H-2′), 2.00(m, 1, H-2′). CMR (75.46 MHz; $D_2O$): 168.44(C-4), 153.69(C-2), 144.07(C-6), 103.71(C-5), 87.79 + 85.97(C-1′ + C-4′), 64.16 + 63.89(C-5′ + C-6′), 42.06(C-2′), 36.57(C-3′). UV(nm): 206(7195), 264(8033).

## Example 13

1-[5-O-Benzyl-3-deoxy-3-[(benzyloxy)methyl]-β-D-ribofuranosyl]-4-thiouracil.

1-[5-O-Benzyl-3-deoxy-3-[(benzyloxy)methyl]-β-D-ribofuranosyl]uracil (480 mg; 1.095 mmol) produced in Example 10 was dissolved in dry 1,2-dimethoxyethane and treated with Lawesson's reagent (480 mg; 1.186 mmol). Chromatographic purification on silica (20% ethyl acetate to 50% ethyl acetate in hexane) yielded pure product (385 mg; 84.7% yield). PMR(300 MHz, $CDCl_3$): 7.91(d, 1, H-6), 7.40 -7.20(m, 10 aromatic), 6.03(m, 2, H-1′ + H-5), 4.54(bs, 2, benzyl $CH_2$), 4.48(bs, 2, benzyl $CH_2$), 4.07 (m, 1, H-4′), 3.90-(dd, 1, H-5′), 3.64 - 3.40(m, 3, H-5′ + 2 x H-6′), 2.67(m, 1, H-3′), 2.28(m, 1, H-2′), 2.12(m, 1, H - 2′).

## Example 14

1-[5-O-Benzyl-3-deoxy-3-[(benzyloxy)methyl-β-D-ribofuranosyl] cytosine.

1-[5-O-Benzyl-3-deoxy-3-[(benzyloxy)methyl-β-D-ribofuranosyl]4-thiouracil (345 mg; 0.787 mmol) was dissolved in ethanol (5 mL) and saturated with ammonia gas. The reaction mixture was heated in a pressure vial at 105°C for 4 h. Chromatography on silica gave the title compound (267 mg; 80.3% yield). PMR(300 MHz, $CDCl_3$): 8.01(d, J = 7.2 Hz, 1, H-6). 7.40 - 7.21(m, 10, aromatic), 6.06(dd, $J_1$ = 6.4 Hz, $J_2$ = 3.0 Hz, 1, H-1′), 5.40(d, J = 7.2 Hz, 1, H-5), 4.53 (narrow AB, 2, benzyl $CH_2$), 4.44(bs, 2, benzyl $CH_2$), 4.01(dm, J = 5.4 Hz, 1, H-4′), 3.87 (dd, $J_1$ = 2.4 Hz, $J_2$ = 10.8 Hz, 1, H-5′), 3.65 - 3.37(m, 3, H-5′ + 2 x H-6′), 2.56-(m, 1, H-3′), 2.25(m, 1, H-2′), 2.10(m, 1, H-2′).

## Example 15

1-[5-O-Benzyl-2,3-dideoxy-3-[(benzyloxy)methyl]-β-D-erythro-pentofuranosyl]uracil and 1-[5-O-benzyl-2,3-dideoxy-3-[(benzyloxy)methyl]-α-D-erythro-pentofuranosyl]uracil.

1-[5-O-Benzyl-2,3-dideoxy-3-[(benzyloxy)methyl]-β-D-erythro-pentofuranosyl]uracil (36 mg; 0.085 mmol) was dissolved in dry acetonitrile (0.8 mL) and treated with dry uracil (22.4 mg; 0.2 mmol), HMDS (42 μL; 0.2 mmol), TMCS (25 μL; 0.2 mmol) and trifluoromethanesulfonic acid (17.7 μL; 0.2 mmol). The reaction mixture was heated at 80°C for 2 h, allowed to cool, diluted with dichloromethane, washed with saturated bicarbonate, brine and dried. Evaporation and subsequent chromatographic purification on silica gel gave a mixture of α-D and β-D nucleosides in the ratio of 2:1 (27 mg; 75% yield). This mixture was not resolved, but used directly for the animation and deprotection sequence.

## Example 16

1,(2,3-Dideoxy-3-C-hydroxymethyl-β-D-erythro-pentofuranosyl)cytosine and 1-(2,3-dideoxy-3-C-hydroxymethyl-α-D-erythro-pentofuranosyl)cytosine.

A 2:1 mixture of 1-[5-O-benzyl-2,3-dideoxy-3-[(benzyloxy)methyl]-β-D-erythro-pentofuranosyl]uracil and 1-[5-O-benzyl-2,3-dideoxy-3-[(benzyloxy) methyl]-α-D-erythro-pentofuranosyl]uracil (260 mg; 0.615 mmol) was dissolved in dry pyridine (5 mL) and treated with 4-chlorophenyl phosphorodichloridate (65 μL; 4.0 mmol) and sublimed triazole (553.6 mg; 8.0 mmol). The reaction mixture was stirred under argon at room temperature for 16 h, dioxane (10 mL), methanol (10 mL) and concentrated ammonia (1 mL) were then added. Stirring was continued for 24 h, the majority of the volatiles was removed in vacuo and the residue was partitioned between ethyl acetate and water. The ethyl acetate fraction was chromatographed on silica gel to give 2:1 mixture of 1-[5-O-benzyl-2,3-dideoxy-3-[(benzyloxy)methyl]-β-D-erythro-pentofuranosyl]-cytosine and 1-[5-O-benzyl-2,3-dideoxy-3-[(benzyloxy)methyl]-α-D-erythro-pentofuranosyl]cytosine (140 mg; 54% yield).

The above mixture was dissolved in ethanol (5 mL), cyclohexene (3 mL) and $Pd(OH)_2/C$ (355 mg) and heated under reflux for 4 h. Volatiles were removed in vacuo and the residue filtered through silica to give 2:1 mixture of 1-(2,3-dideoxy-3-C-hydroxymethyl-β-D-erythro-pentofuranosyl)cytosine and 1-(2,3-dideoxy-3-C-hydroxymethyl-α-D-erythro-pentofuranosyl)cytosine, which was separated through HPLC on a $C_{18}$ column (0 - 3% methanol in 0.05 M ammonium formate). Final freeze-drying with a few drops of concentrated ammonia.

1-(2,3-Dideoxy-3-C-hydroxymethyl-α-D-erythro-pentofuranosyl)cytosine was eluted first (10 mg; 6.7% yield) PMR(300 MHz; $CD_3OD$): 7.82 (d, J = 7.6 Hz, 1, H-6), 6.05(t, J= 6.4 Hz, 1, H-1′), 5.95(bd, J = 7.5 Hz, 1, H-5), 4.19 (m, 1, H-4′), 3.75(dd, $J_1$ = 3.6 Hz, $J_2$ = 12.1 Hz, 1, H-5′), 3.62 - 3.52(m, 3, H-5′ + 2 x H-6′), 2.65(m, 1, H-2′), 2.41(m, 1, H-3′), 1.84(m, 1, H-2′). CMR(75.47 MHz; $D_2O$): 173.9(C-4), 164.7(C-2), 148.42(C-6), 102.91(C-5), 94.37(C-1′), 90.52(C-4′), 69.62 + 68.71(C-5′ + C-6′), 48.11(C-3′), 42.25(C-2′). MS-high resolution (TAB in glycerol): For $C_{10}H_{16}O_4N_3$ calc: 242.1141; Found: 242.1133.

1-(2,3-Dideoxy-3-C-hydroxymethyl-β-D-erythro-pentofuranosyl)cytosine eluted second (21 mg; 14.15% yield). M.p. 171 - 174°C. PMR(300 MHz; $D_2O$): 7.86(d, J = 7.6 Hz, 1, H-6), 6.035(dd, $J_1$ = 4.0 Hz, $J_2$ = 6.9 Hz, H-1′), 5.93(d, J = 7.5 Hz, 1, H-5), 3.95(m, 1, H-4′), 3.86(dd, $J_1$ = 2.9 Hz, $J_2$ = 12.6 Hz, 1, H-5′), 3.70(dd, $J_1$ = 5.4 Hz, $J_2$ = 12.6 Hz, 1, H-1′), 3.62(d, J = 5.7 Hz, 2, H-6′), 2.41 - 2.10(m, 3, H-3′ + 2 x H-2′). CMR(75.47 MHz; $D_2O$): 173.2(C-4), 164.6(C-2), 148.6(C-6), 102.8(C-5), 93.3 + 91.0(C-1′ + C-4′), 69.22 + 68.84(C-5′ + C-6′), 46.95(C-3′), 42.2(C-2′). UV($H_2O$): 272(4921). MS - high resolution (FAB in glycerol): For $C_{10}H_{16}N_3O_4$ calc.: 242.1411; found: 242.1139.

## Example 17

5-O-Benzyl-3-deoxy-3-[(benzyloxy)methyl]-1,2-anhydro-α-D-ribofuranose.

1,2-O-Isopropylidene-3-deoxy-3-[(benzyloxy)methyl]-5-O-benzyl-α-D-ribofuranose (350 mg; 0.91 mmol) dissolved in chloroform (10 mL) was heated under reflux for 36 h with 10% ferric chloride/silica. The solids were filtered off, solvent was evaporated and the residue was purified on silica column (30% ethyl acetate in

hexane) to give the title product as an oil (230 mg; 77.4% yield). PMR(360 MHz; $CDCl_3$): 7.34 - 7.22(m, 10, aromatic), 5.06(d, J = 3.75 Hz, 1, H-1′), 4.55(S, 2, benzyl $CH_2$), 4.45(AB, 2, benzyl $CH_2$), 4.27(m, 1, H-4′), 3.93 (dd, $J_1$ = 6.6 Hz, $J_2$ = 3.8 Hz, 1, H-2′, 3.82(m, 2, H-6′ + H-5′), 3.53(m, 2, H-5′ + H-6′), 2.52(m, 1, H-3′). CMR(75.47 MHz; $CDCl_3$): 138.89 + 138.40 (quaternary aromatic), 128.97 - 128.18 (tertiary aromatic), 99.45(C-1′), 82.2(C-4′), 73.91 + 73.71 + 73.44 (C-2′ + 2 x benzylic $CH_2$), 70.80 + 67.07(C-5′ + C-61), 44.43(C-3′). MS-FAB(glycerol matrix): 327 ($M^+$ + H).

## Example 18

1-[5-O-Benzyl-3-deoxy-3-[(benzyloxy)methyl]-β-D-ribofuranosyl]thymine.

A solution of persilylated thymine (0.9 mmol) and 5-O-benzyl-3-deoxy-3-[(benzyloxy)methyl]-1,2-anhydro-α-D-ribofuranose (100 mg; 0.306 mmol) in 1,2-dichloroethane/dichloromethane (1:1; 4 mL) was treated with trimethylsilyl trifluoromethanesulfonate (193.3 µL; 1.0 mmol). After 3 h stirring at room temperature under argon, the reaction mixture was diluted with dichloromethane (10 mL) and washed with cold bicarbonate and water. After drying and evaporation, the crude material (100 mg) was purified on a silica gel column (30% to 60% ethyl acetate in hexane). Only partial purification was achieved. The nucleoside fractions were purified further by HPLC on a µPorasil column (5% iPrOH in methylene chloride) to give the title product (11 mg; 7.9% yield). PMR(360 MHz; $CDCl_3$): 7.87(s, 1, H-6), 7.36 - 7.24(m, 10, aromatic), 5.76(s, 1, H-1′), 4.56 (AB, 2, benzyl $CH_2$), 4.50(s, 2, benzyl $CH_2$), 4.36(m, 2, H-2′ + H-4′), 3.98(D, J = 9.65 Hz, 1, H-6′), 3.775(dd, $J_1$ = 6.05, $J_2$ = 9.5 Hz, 1, H-5′), 3.63(m, 2, H-5′ + H-6′), 2.62(m, 1, H-3′), 1.50 (bs, 3, $CH_3$) . CMR(75.47 MHz; $COCl_3$): 164.6(C-4), 151.9(C-2), 138.3 + 138.0 + 136.9 (quaternary aromatic + C-6), 129.14 - 128.20 (tertiary aromatic), 110.49(C-5), 93.023(C-1′), 83.64(C-4′), 77.72(C-2′), 73.97 + 73.81(benzyl $CH_2$), 69.54 + 66.88(C-5′ + C-6′), 40.88(C-3′), 12.08($CH_3$). MS-FAB(glycerol matrix): 453$M^+$ + H).

## Example 19

5′-O-Benzyl-3′-deoxy-3′-[(benzyloxy)methyl]-2,2′-anhydro-β-D-uridine.

1-[5-O-Benzyl-3-deoxy-3-[(benzyloxy]methyl]-β-D-ribofuranosyl]uracil (11.0 g; 25.1 mmol) and 1,1′-thiocarbonyldiimidazole (5.377 g; 30.1 mmol) were dissolved in dry 1,2-dichloroethane (200 mL) and heated under reflux for 40 minutes. The volatiles were removed in vacuo and the remaining material was dissolved in dry THF (45 mL). This solution was added dropwise over 40 minutes to a solution of tributyltin hydride (40.35 mL; 43.65 g; 140 mmol) and AIBN (500 mg; 3.05 mmol) in dry toluene (250 mL), stirred vigorously under argon and heated to reflux. Heating was continued for three h. The volatiles were removed in vacuo and the residue was purified on silica gel column. 1-[5-O-benzyl-2,3-dideoxy-3-[(benzyloxy)methyl-β-D-ribofuranosyl]uracil was eluted first (2.0 g; 18.9%) with 4% of ethanol in 1:1 mixture of ethyl acetate and hexane. 5′-O-benzyl-3′-deoxy-3-[(benzyloxy)methyl]-2,2′-anhydro-β-D-uridine was eluted with 50% of ethanol in ethyl acetate (8.0 g; 75.1%).

## Example 20

1-[5-O-Benzyl-3-deoxy-3-[(benzyloxy)methyl]-β-D-arabinofuranosyl]uracil.

5′-O-Benzyl-3′-deoxy-3′-[(benzyloxy)methyl-2,2′-anhydro-β-D-uridine (2.0 g; 4.76 mmol) was dissolved in ethanol (100 mL) and heated under reflux with 1N sodium hydroxide for 2 h. Adjustments of pH to 7.0 with 80% acetic acid (cooling in water - ice) led to precipitation of the title compound, which was collected and dried (1.34 g; 65.2%). PMR(300 MHz; $CDCl_3$) : 7.35 - 7.16(m, 11, aromatic + H-6), 6.02(bd, J = 6.3

Hz, 2, H-1' + H-5), 5.24(d, J = 6.0 Hz, 1, H-2'), 4.49 (narrow AB, 2, benzylic $CH_2$), 4.41 (A of AB, J = 12.6 Hz, 1, benzylic $CH_2$), 4.28(m, 1, H-4'), 4.26(B of AB, J = 12.0 Hz, 1, benzylic $CH_2$), 3.62 - 3.57 and 3.47 -3.42(AB of ABX, 2, $CH_2OBn$), 3.36 - 3.31 and 3.23 - 3.18 (AB of ABX, 2, $CH_2OBn$), 2.78(m, 1, H-3'). CMR-(75.47 MHz; $CDCl_3$) : 173.44(C-4), 161.07(C-2), 137.93 + 137.61 (quaternary aromatic), 135.74(C-6), 129.183 + 129.05 + 128.93 + 128.76 + 128.64 + 128.59 + 128.44 + 128.27 (aromatic), 110.35(C-5), 91.70(C-1'), 86.79(C-4'), 84.63(C-2'), 73.97 + 73.76(benzylic $CH_2$), 70.64 + 69.75(C-5' + C-6'), 47.95 (C-3').

## Example 21

1-[2,5-Di-O-acetyl-3-deoxy-3-[(acetoxy)methyl-β-D-arabinofuranosyl]-4-thiouracil.

1-[5-O-Benzyl-3-deoxy-3-[(benzyloxy)methyl]-β-D-arabinofuranosyl]uracil (647 mg; 1.47 mmol) was dissolved in a mixture of ethanol (5 mL) and cyclohexene (5 mL) and refluxed for 4 h with Pearlman's catalyst (1.0 g). The catalyst was filtered off (Celite) and the volatiles removed. The crude product (390 mg) was sufficiently pure for subsequent steps. PMR(300 MHz; DMSO-$d_6$): 7.75(d, J = 8.1 Hz, 1, H-6), 5.85(d, J = 5.1 Hz, 1, H-1'), 5.51(d, J = 8.1 Hz, 1, H-5), 5.38(bd, 1, OH), 5.0(bs, 1, OH), 4.8(bs, 1, OH), 4.14(m, 1, H-2'), 3.74 (m, 1, H-4'), 3.70 - 3.45(m, 4, 2 x $CH_2OH$), 2.02(m, 1, H-3'). CMR(75.47 MHz; $CD_3OD$): 167.25(C-4) 153.1(C-2), 144.95(C-6), 101.46(C-5), 87.74 (C-1'), 82.39(C-4'), 74.21 (C-2'), 63.98 + 61.83(C-5') + C-6'), 50.57 (C-3'). 1-(3-Deoxy-3-C-hydroxymethyl-β-D-arabinofuranosyl)uracil (390 mg) was acetylated with acetic anhydride (1 mL) and pyridine (2 mL) overnight to give the triacetate used crude for the next step. PMR(300 MHz; $CDCl_3$) : 7.48(d, J = 8.1 Hz, 1, H-6), 6.10(d, J = 4.5 Hz, 1, H-1'), 5.68(d, J = 8.1 Hz, 1, H-5), 5.30(bt, J = 5.1 Hz, 1, $CH_2OAc$), 4.05(m, 1, H-4'), 2.47(m, 1, H-3'), 2.06 + 2.03 + 1.93(3xs, 3, $CH_3CO$). This material (320 mg; 0.83 mmol) was dissolved in dry 1,2-dimethoxyethane (10 mL) and treated with Lawesson's reagent (660 mg; 1.33 mmol) at 50°C for 12 h. The volatiles were removed in vacuo and the residue was purified on silica gel column using dichloromethane to 40% ethyl acetate in dichloromethane. Yield: 265 mg (79.5%). PMR(300 MHz; $CDCl_3$): 7.37(d, J = 7.5 Hz, 1, H-6), 6.40(d, J = 7.5 Hz, 1, H-5), 6.08(d, J = 4.8 Hz, 1, H-1'), 5.37(t, J = 4.8 Hz, 1, H-2'), 4.38 - 4.25(m, 2, H-5'), 4.21(m, 2, H-6'), 4.10(m, 1, H-4'), 2.50(m, 1, H-3'), 2.10(s, 3, $OCOCH_3$), 2.07(s, 3, $OCOCH_3$), 1.99(s, 3, $OCOCH_3$). CMR(75.47 MHz; $CDCl_3$) : 190.47(C-4), 171.54 + 171.39 + 170.14 (3 x $CH_3CO$), 148.22(C-2), 135.64(C-6), 113.27(C-5), 85.1 (C-1'), 77.95(C-4'), 74.25(C-2'), 64.46 + 62.39(C-5' + C-6'), 45.31 (C-3'), 21.02 + 20.92 + 20.84 (3 x $CH_3CO$).

## Example 22

1-(3-Deoxy-3-C-hydroxymethyl-β-D-arabinofuranosyl)cytosine.

A stainless steel bomb (100 mL) was charged with a solution of 1-(2,5-di-O-acetyl-3-deoxy-3-[acetoxymethyl]-β-D-arabinofuranosyl]4-thiouracil (210 mg; 0.52 mmol) in methanol (20 mL) saturated with ammonia gas at 0 - 5°C and heated at 100 - 110°C. The volatiles were removed in vacuo and the residue was purified on silica using 10% methanol in dichloromethane to 50% methanol in dichloromethane. Main fractions were crystallized from ethanol - diethyl ether. M.p.: 179 - 183°C. PMR(300 MHz; $CD_3OD$): 7.71(d, J = 7.4 Hz, 1, H-6), 5.79(d, J = 4.8 Hz, 1, H-1'), 5.64(d, J = 7.6 Hz, 1, H-5), 4.10 (bt, J = 4.6 Hz, 1, H-2'), 3.70 (m, 1, H-4'), 3.65 - 3.45(m, 4, 2 x $CH_2OH$), 1.97(m, 1, H-3'). CMR(75.47 MHz; $CD_3OD$) : 168.21(C-4), 159.27(C-2), 145.07(C-6), 95.44(C-5), 88.55(C-1'), 82.25(C-4'), 74.10(C-2'), 64.24 + 62.07(C-5' + C-6'), 50.98(C-3'). MS-High Resolution (FAB in glycerol matrix): for $C_{10}H_{16}N_3O_5$ Calc.: 258.1090; Found: 258.1084.

## Example 23

9-[5-O-Benzyl-3-deoxy-3-[(benzyloxy)methyl]-β-D-ribofuranosyl]adenine.

1,2-Di-O-acetyl-5-O-benzyl-3-deoxy-3-[(benzyloxy)methyl-β-D-ribofuranose (2.01 g; 4.7 mmol; 30% of the α-isomer) and N-benzoyl-chloromercuriadenine (2.79 g; 5.89 mmol) in dry 1,2-dichloroethane (200 mL) were heated under reflux (vigorous stirring, argon) with Celite (2.8 g) and titanium chloride (0.65 ml; 5.93 mmol). After 18 h the mixture was allowed to cool and treated with saturated bicarbonate solution (15 mL). Stirring was continued for 2 h and the mixture was filtered through Celite. The organic layer was concentrated, taken up in chloroform and washed with 30% potassium iodide solution, water and dried. The crude material (2.14 g) contained about 46% of the desired product by $^1$H. Column chromatography on silica gel yielded 9-[2-O-acetyl-5-O-benzyl-3-deoxy-3[(benzyloxy)methyl]-β-D-ribofuranosyl]N-benzoyladenine (747 mg; 25.5%), which was eluted with 40% of ethyl acetate in dichloromethane. PMR(300 MHz; $CDCl_3$;) : 9.33 (bs, 1, NHBz), 8.75(s, 1, H-8), 8.47(s, 1, H-2), 7.98(d, 2, N-benzoyl), 7.50(m, 1, N-benzoyl), 7.43(m, 2, N-benzoyl), 7.32 - 7.20(m, 10, benzyl aromatic), 6.21 (d, J = 1.8 Hz, 1, H-1′), 5.73(dd, $J_1$ = 1.8 Hz, $J_2$ = 5.7 Hz, 1, H-2′), 455(broad AB, 2, benzylic $CH_2$), 4.46 (narrow AB, 2, benzylic $CH_2$), 4.36(m, 1, H-4′), 3.88 -3.84 and 3.42 - 3.38 (AB of ABX, 2, $CH_2OBn$), 3.41 - 3.36 and 3.52 - 3.47 (AB of ABX, 2, $CH_2OBn$), 3.14(m, 1, H-3′), 2.02(s, 3, $CH_3CO$). This material (600 mg; 0.96 mmol), was dissolved in methanol (40 mL) and the solution was saturated with ammonia gas at 0° C. This solution was placed in a steel bomb (100 mL) and heated at 130° 6 (bath) for 4 h. Volatiles were removed in vacuo to give the desired product (440 mg; quant.) . PMR(300 MHz; $CDCl_3$): 8.28 + 8.29(2xs, 1, H-8 + H-2), 7.33 - 7.20(m, 10, benzyl aromatic), 6.0(d, H = 1.8 Hz, 1, H-1′), 5.63 (Bs, 2, $NH_2$), 4.66(m, 1, H-2′), 4.55 (broad AB, 2, benzylic $CH_2$, 4.50 (narrow AB, 2, benzylic $CH_2$), 4.47(m, 1, H-4′), 3.88 - 3.84 and 3.59 - 3.55 (AB of ABX, 2, $CH_2OBn$), 3.70(m, 2, $CH_2OBn$), 2.78(m, 1, H-3′).

## Example 24

9-[5-O-Benzyl-2,3-dideoxy-3-[(benzyloxy)methyl-β-D-ribofuranosyl]adenine.

9-[5-O-Benzyl-3-deoxy-3-[(benzyloxy)methyl]-β-D-ribofuranosyl]adenine (440 mg; 0.95 mmol) and thionocarbonylimidazone (229.8 mg; 1.287 mmol) were heated under reflux in dry 1,2-dichloroethane (25 mL) for 1 h. Volatiles were removed in vacuo and the residue (645 mg) dissolved in dry THF (5 mL) was added dropwise to a refluxing solution of tributyltin hydride (1.5 mL; 5.58 mmol) and azobisisobutyronitrile (AIBN; 50 mg) in dry toluene (20 mL). Reflux was continued for 8 h, the volatiles were removed in vacuo and the residue was purified on a silica gel column using 30% ethyl acetate in dichloromethane to pure ethyl acetate to give the title product (250 mg; 59%). PMR(300 MHz; $CDCl_3$): 8.31 + 8.24 (2 x s, 1, H-8 + H-2), 7.35 - 7.20(m, 10, benzyl aromatic), 6.32(dd, $J_1$ = 3.0 Hz, $J_2$ = 6.6 Hz, 1, H-1′), 5.51(bs, 2, $NH_2$), 4.57 (broad AB, 2, benzylic $CH_2$), 4.48(s, 2, benzylic $CH_2$), 4.13(m, 1, H-4′), 3.82 - 3.77 and 3.67 -3.61 (AB of ABX, 2, $CH_2OBn$), 3.50(d, J = 6.3 Hz, 2, $CH_2OBn$), 2.81(m, 1, H-3′), 2.59 - 2.51(m, 1, H-2′), 2.44 - 2.37-(m, 1, H-2′).

## Example 25

9-[2,3-Dideoxy-3-C-hydroxymethyl-β-D-erythro-pentofuranosyl]adenine.

9-[5-O-Benzyl-2,3-dideoxy-3-[(benzyloxy)methyl]-β-D-ribofuranosyl]adenine (800 mg; 1.80 mmol) dissolved in a mixture of ethanol (25 mL) and cyclohexene (35 mL) was heated under reflux for 9 h with Pearlman's catalyst (3.85 g). The reaction mixture was filtered through Celite and the volatiles removed under vacuo. The residue was purified on silica gel using ethyl acetate to 50% ethanol in ethyl acetate and the fractions containing the main product were crystallized from ethanol to give the title product (87 mg; 18.12%). M.p. 171 - 174° C. PMR(300 MHz; $D_2O$): 8.30 + 8.16(2 x s, 1, H-8 + H-2), 6.31(dd, $J_1$ = 2.7 Hz, $J_2$ = 6.6 Hz, 1, H-1′), 4.1(m, 1, H-4′), 3.84 - 3.79 and 3.66 -3.60(AB of ABX, 2, $CH_2OH$), 3.71(d, J = 5.5 Hz, 2, $CH_2OH$), 2.66(m, 2, H-3′ + H-2′), 2.45(m, 1, H-2′). CMR(75.47 MHz; $CD_3OD$) : 154.18(C-2), 141.74 (C-8), 87.17 + 86.26 (C-1′ + C-4′), 64.55 + 63.84(C-5′ + C-6′), 42.58(C-3′), 37.14(C-2′). MS high resolution;

(FAB in glycerol matrix): for $C_{11}H_{16}N_6O_3$: Calc.: 266.1253; Found: 266.1256. IR(KBr; $cm^{-1}$) : 3700 - 3000, 1645. UV($H_2O$; 1.46 x $10^{-5}$) : 260 (13130). For $C_{11}H_{15}N_5O_3$ x $H_2O$: Calc.: 46.64% C; 6.05% H; 24.72% N; Found: 47.11% C; 5.53% H; 24.69% N.

## Example 26

9-(2,3-Dideoxy-3-C-hydroxymethyl-β-D-erythro-pentofuranosyl)hypoxanthine.

9-(2,3-Dideoxy-3-C-hydroxymethyl-β-D-erythro-pentofuranosyl)adenine (40 mg; 0.015 mmol) in water (2 mL) was incubated overnight with adenosine deaminase (1 mg, SIGMA). The product was purified by HPLC on IBSil $C_{18}$ column using 2-5% methanol in 0.025 M ammonium formate and crystallized from ethanol. Yield: 16 mg (38%). M.p.>250°C. Dec.>190°C. PMR(30 MHz; $D_2O$) : 8.32 + 8.17(2xs, 1, H-2 + H-8), 6.36(dd, $J_1$ = 3.0 Hz, $J_2$ = 6.6 Hz, 1, H-1′), 4.09(m, 1, H-4′), 3.83 - 3.78 and 3.67 - 3.62(AB of ABX, 2, $CH_2OH$), 3.71(d, J = 5.5 Hz, 2, $CH_2OH$), 2.66(m, 2, H-3′ + H-2′), 2.44(m, 1, H-2′). CMR(75.47 MHz; $CD_3OD$) : 147.20(C-2), 141.04(C-8), 87.04 + 86.41(C-1′ + C-4′), 64.26 + 63.62(C-5′) + C-6′), 42.42(C-3′), 37.42(C-2′). MS high resolution (FAB in glycerol matrix): for $C_{11}H_{15}N_4O_4$ Calc.: 267.1093; Found: 267.1091. UV($H_2O$; 4.49 x $10^{-5}$M): 250(9590).

## Example 27

5-O-Benzyl-1,2-O-isopropylidene-α-D-xylofuranose.

To 1.2 isopropylidene xylofuranose (10.0 g, 52.6 mmol) was added dibutyltin oxide (13.1 g, 52.6 mmol) in 300 mL of benzene. The solution was warmed to reflux for 6 h with azeotropic removal of water. The solution was concentrated to 100 mL and tetrabutyl ammonium iodide (4.7 g, 12.7 mmol) and benzyl bromide (120 mL, 168.4 mmol) were added. The solution was returned to reflux and held for 72 h. The solution was cooled, poured into sat $NaHCO_3$ and extracted with chloroform. The organic fraction was dried over $MgSO_4$, filtered and concentrated. The residue was chromatographed over silica gel (3:1 hexane/ethyl acetate) and concentrated to provide 10.7 g of the monobenzyl ether as a waxy white solid (72%); PMR(300 MHz, $CDCl_3$) δ 7.31(m, 5H, aromatic), 5.95(d, J = 3.7 Hz, 1H, H-1′), 4.58(ABq, J = 12.7, 12.0 Hz, 2H, benzyl), 4.48(D, J = 3.5 Hz, 1H, H-2′), 4.26(m, 1H, H-4′), 4.22(m, 1H, H-3′), 3.90(m, 2H, H-5′), 3.56(d, J = 3.3 Hz, 1H, OH), 1.44(s, 3H, $CH_3$), 1.28(s, 3H, $CH_3$); CMR(75.5MHz) δ 137.8, 129.1, 128.6, 128.4, 112.1, 105.4, 85.8, 78.6, 76.7, 74.4, 68.6, 27.0, 26.4.

## Example 28

5-O-Benzyl-1,2-O-isopropylidene-α-D-erythro-pentofuranose-3-ulose.

A solution of DMSO (2.5 mL, 35.2 mmol) in 15 mL of methylene chloride was cooled to -78°C and trifluoroacetic anhydride was added (3.6 mL, 0.36 M in $CH_2Cl_2$, 3.6 mmol) over 10 min. A solution of the benzyl ether of Example 27 (4.8 g, 1.7M in $CH_2Cl_2$, 17.1 mmol) was added at -78°C and stirred for 30 min. Finally, triethylamine (7.1 mL, 50.9 mmol) was added in one portion at -78°C, the cold bath removed, and the solution brought to ambient temperature. The solution was diluted with ether and washed with 10% HCl, saturated $NaHCO_3$, and brine. The organic fraction was dried over $MgSO_4$ and concentrated to provide 4.4 g (92%) of a yellow oil which was not purified: PMR(300 MHz, $CDCl_3$) δ 7.25(m, 5H, aromatic), 6.10(d, J = 4.4 Hz, 1H, H-1′), 4.48(AB, J = 2.7 Hz, 2H, benzyl), 4.42(m, 1H, H-4′), 4.31 (dd, J = 4.4, 1.1 Hz, 1H, H-2′), 3.70 (AB, J = 2.4 Hz, 2H, H-5′), 1.43(s, 3H, $CH_3$), 1.40(s, 3H, $CH_3$); CMR(75.5 MHz) δ 138.0, 129.0, 128.5, 128.1, 114.7, 104.1, 80.3, 77.2, 74.1, 70.5, 27.8, 27.2.

Example 29

5-O-Benzyl-1,2-O-isopropylidene-3-deoxy-3-C-methylene-α-D-ribofuranose.

To a suspension of methyltriphenylphosphonium bromide (189 g, 0.529 mol) in 1 L of anhydrous ether at 0° C was added n-butyl lithium(200 mL, 2.5 M in hexane, 0.50 mmol). To the orange solution of the ylide was added the crude ketone prepared in Example 28 (48 g, 0.172 mol) in 250 mL of anhydrous ether. After 30 min at 0° C, the solution was quenched with water, the organic layer separated and dried over $MgSO_4$. The etheral solution was filtered through a pad of Celite, concentrated and diluted with pentane and refiltered to give 34.5 g (72%) of the alkene: PMR(300 MHz, $CDCl_3$) δ 7.30(m, 5H, aromatic), 5.85(d, J = 4.0 Hz, 1H, H-1′), 5.39(m, 1H, vinyl), 5.16(m, 1H, vinyl) 4.86(m, 2H, H-3′ and H-4′), 4.56 (s, 2H benzyl), 3.64(dd, J = 10.3, 3.8 Hz, 1H, H-5'a), 3.53(dd, J = 10.3, 4.8 Hz, 1H, H-5'b), 1.47(s, 3H, $CH_3$), 1.35(s, 3H, $CH_3$); CMR (75.5 MHz) δ 147.5, 138.6, 129.0, 128.2, 112.9, 112.3, 105.2, 82.2, 79.1, 73.8, 72.1, 27.6, 27.4.

Example 30

5-O-Benzyl-1,2-O-isopropylidene-3-deoxy-3-C-hydroxy-methyl-α-D-ribopentofuranose.

To a solution of $BH_3$-THF (19 mL, 1.0M in THF, 19 mmol) at 0° C was added a solution of the alkene produced in Example 29 in 20 mL of THF (2.7 g, 9.8 mmol) and kept in the cold for 3 h. The solution was quenched with 17 mL of a 1:1 mixture of THF and water and then stirred with 20 mL of 2N NaOH and 16 mL of 30% $H_2O_2$ for 1h. The organic fraction was separated and washed with sat $Na_2S_2O_3$, dried over $MgSO_4$, and concentrated. The residue was chromatographed over silica gel (2:1 hexane/ethyl acetate, then 1:1 hexane/ethyl acetate) to give 1.6 g of the hydroxymethyl adduct as a white solid (55%): mp 73-74° C; PMR(300 MHz, $CDCl_3$ δ 7.30(m, 5H, aromatic), 5.79(d, J = 3.7 Hz, 1H, H-1′), 4.73(t, J = 4.3 Hz, 1H, H-2′), 4.56(ABq, J = 11.9, 9.8 Hz, 2H, benzyl), 4.20(m, 1H, H-4′), 3.80(m, 2H, H-6′), 3.65(dd, J = 10.2, 4.3 Hz, 1H, H-5′a), 3.59 (dd, J = 10.2, 5.2 Hz, 1H, H-5′b), 2.70(brs, 1H, OH), 2.15(m, 1H, H-3′), 1.48(s, 3H, $CH_3$), 1.29(s, 3H, $CH_3$) ; CMR(75.5 MHz) δ 138.2, 129.0, 128.4, 112.4, 105.4, 82.1, 79.3, 74.0, 70.6, 59.5, 48.6, 26.8, 26.4.

Example 31

5-O-Benzyl-3-deoxy-3-[(benzoyloxy)methyl]-1,2-O-isopropylidene-α-D-ribofuranose.

To a solution of the primary alcohol produced in Example 30 (900 mg, 3.05 mmol) in 10 mL of pyridine was added benzoyl chloride (1.5 g, 10.7 mmol). The solution was stirred 24 h at ambient temperature and then diluted with ether and washed with saturated $NaHCO_3$ solution. The organic fraction was dried over $MgSO_4$, concentrated under reduced pressure and the residue chromatographed over silica gel (3:1 hexane/ethyl acetate) to provide 1.1 g (89%) of a clear oil: PMR(300.1 MHz, $CDCl_3$) δ 8.00(m, 2H, aromatic), 7.56(m, 1H, aromatic), 7.42(m, 2H, aromatic), 7.29(m, 5H, aromatic), 5.88(d, J = 3.7Hz, 1H, H-1′), 4.78(t, J = 4.2 Hz, 1H, H-2′), 4.59(dd, J = 11.1, 7.6 Hz, 1H, H-6′a), 4.53(ABq, J = 12.2, 8.3 Hz, 2H, benzyl), 4.41-(dd, J = 11.1, 6.6 Hz, 1H, H-6′b), 4.16(dt, J = 10.2, 4.1 Hz, 1H, H-4′), 3.80(dd, J = 10.9, 2.9 Hz, 1H, H-5′a), 3.60(dd, J = 10.9, 4.4 Hz, 1H, H-5′b), 2.58(m, 1H, H-3′), 1.52(s, 3H, $CH_3$), 1.34(s, 3H, $CH_3$) ; CMR-(75.5 MHz) δ 167.0, 138.6, 134.2, 133.7, 130.7, 130.2, 129.0, 128.2, 112.6, 105.6, 81.3, 79.6, 74.0, 70.1, 61.7, 44.6, 27.0, 26.6. Anal. Calcd. for $C_{23}H_{26}O_6$; C, 69.33; H, 6.58. Found: C, 69.33, H, 6.77.

Example 32

1,2-Di-O-acetyl-5-O-benzyl-3-deoxy-3-[(benzoyloxy)methyl]-α- and β-D-ribofuranose.

A solution of the 1,2 acetonide produced in Example 31 13.5 g, 8.6 mmol) in 29 mL of 65% acetic acid (19 mL AcOH/10 mL water) was heated to 100-110° C for 6 h. The solution was cooled and the volatiles were removed under reduced pressure and the residue dissolved in 30 mL of pyridine and stirred 12 h with 5 mL of acetic anhydride. The volatiles were removed under reduced pressure and the residue chromatographed over silica gel to afford 3.7 g (95%) of a clear oil in which the β anomer predominated: PMR(β anomer) (300.1 MHz, $CDCl_3$) δ 7.98(m, 2H, aromatic), 7.59(m, 1H, aromatic), 7.43(m, 2H, aromatic), 7.32(m, 5H, aromatic), 6.11 (s, 1H, H-1′), 5.38(d, J = 4.8 Hz, 1H, H-2′), 4.56 (ABq, J = 5.9, 2.3 Hz, 2H, benzyl), 4.47(dd, J = 6.6, 2.3 Hz, H-6′a), 4.31(m, 1H, H-4′), 3.66(m, 2H, H-5′), 2.99(m, 1H, H-3′), 2.09(s, 3H, acetate), 1.95(s, 3H, acetate); CMR (75.5 MHz) δ 170.5, 170.0, 166.8, 138.6 133.9, 130.2, 129.1, 129.0, 128.3, 128.2, 99.4, 82.2, 77.0, 73.7, 71.8, 61.0, 41.6, 21.2, 20.9. Anal. Calcd. for $C_{24}H_{26}O_8$; C, 65.15; H, 5.92. Found: C, 65.06; H, 6.16.

## Example 33

1-(2-O-Acetyl-5-O-benzyl-3-deoxy-3-[(benzoyloxy)methyl-β-D-ribofuranosyl)thymine.

Thymine (840 mg, 6.66 mmol) and the 1,2-diacetate produced in Example 32 (2.52 g, 5.60 mmol) were added to 75 mL of acetonitrile. To this solution was added HMDS (1.4 mL, 6.6 mmol) and TMSC (0.85 mL, 6.8 mmol) followed by $SnCl_4$ as a solution in 25 mL of acetonitrile (0.9 mL, 7.7 mmol). The solution was stirred at ambient temperature for 4 h. The solution was diluted with $CNCl_3$ and washed with saturated $NaHCO_3$ and then brine. The organic fraction was dried over $MgSO_4$, concentrated under reduced pressure to give 2.6 g of a white solid (91%) which was recrystallized from hexane/ethyl acetate; mp 129-130° C; PMR(300 MHz, $CDCl_3$) δ 8.19(s, 1H, NH), 8.01(m, 2H, aromatic), 7.58(m, 2H, aromatic and H-6), 7.44(m, 2H, aromatic), 7.28(m, 5H, aromatic), 6.02(d, J = 2.8 Hz, 1H, H-1′), 5.57(dd, J = 6.5, 2.9 Hz, 1H, H-2′), 4.58-(ABq, J = 11.8, 1.1 Hz, 2H, benzyl), 4.47(dd, J = 11.5, 6.5 Hz, 1H, H-6′a), 4.37(m, 2H, H-6′b and H-4′), 3.98 (dd, J = 12.9, 2.0 Hz, 1H, H-5′a), 3.67(dd, J = 11.0, 2.4 Hz, 1H, H-5′b), 3.12(m, 1H, H-3′), 2.10(s, 3H, acetate), 1.56(m, 3H, C-5 methyl); CMR(75.5 MHz) δ 170.3, 166.9, 164.9, 151.2, 137.9, 136.3, 134.0, 130.2, 130.0, 129.2, 128.7, 128.3, 111.5, 89.6, 82.1, 77.1, 74.0, 70.0, 61.1, 40.3, 20.9, 12.6; MS(DCI) m/e 509(M + 1), 449($M^+$ - OAc), 383($M^+$ - thymine). HRMS calcd for $C_{27}N_{28}N_2O_8$ m/e 508.1846. Found: Anal. Calcd. for $C_{27}H_{28}N_2O_8$ - 0.25 $H_2O$; C, 63.21; H, 5.50; N, 5.46. Found: C, 63.34; H, 5.74; N, 5.53.

## Example 34

1-(5-O-Benzyl-3-deoxy-3-[(benzoyloxy)methyl]-2-hydroxy-β-ribofuranosyl)thymine.

A solution of 20 mL of methanol was saturated with ammonia at ambient temperature and then cooled to 0° C. A solution of the 2-acetyl nucleoside produced in Example 33 (2.6 g, 5.1 mmol) in 20 mL of methanol was added to the methanolic ammonia and stirred at 0° C for 2h. The volatiles were removed under reduced pressure and the residue filtered through silica gel (1:2 hexane/ethyl acetate 2% EtOH) to provide 2.3 g (96%) of the deacylated adduct which was recrystallised from ethyl ether, mp 119-120° C: PMR(300.1 MHz, $CDCl_3$) δ 10.26(br s, 1H, NH), 8.02(m, 2H, aromatic), 7.95(s, 1H, H-6), 7.55(m, 1N, aromatic), 7.40(m, 2H, aromatic), 7.25(m, 5H, aromatic), 5.81 (s, 1H, H-1′), 4.74(dd, J = 11.3, 6.7 Hz, 1H, H-6′a), 4.48(m, 4H, H-6′b, H-2′ and benzyl), 4.09(d, J = 10.2 Hz, 1H, H-5′a), 3.71(d, J = 9.7 Hz, 1H, H-5′b), 2.81(m, 1H, H-3′), 1.65(br s, 1H, OH), 1.41(s, 3H, C-5 methyl); CMR(75.5 MHz) δ 167.0, 165.5, 151.5, 138.0, 136.9, 133.8, 130.5, 130.2, 129.2, 129.0, 128.6, 128.3, 110.7, 93.1, 83.5, 77.3, 74.0, 69.2, 61.6, 39.8, 12.0; MS (DCI) m/e 467(M + 1), 449($M^+$ - OH), 341($M^+$ -thymine).

## Example 35

1-(5-O-Benzyl-3(benzyloyloxy)methyl-2,3-dideoxy-β-D-erythro-furanosyl)thymine.

To the 2′-hydroxy nucleoside produced in Example 34 (400 mg, 0.85 mmol) in 10 mL of THF was added N,N-thiocarbonyl diimidazone (225 mg, 1.1 mmol) and the solution warmed to reflux for 3 h. The solution was cooled, concentrated under reduced pressure and the residue chromatographed over silica gel (1:2 hexane/ethyl acetate 10% EtOH). The residue was dissolved in 20 mL of anhydrous, degassed dioxane under nitrogen to which was added 2 mL of polymethylhydrosiloxane, and 10 mg of AIBN. The solution was warned to reflux for 1 h, cooled, and the solution filtered through a pad or Celite. The filtrate was concentrated under reduced pressure and the residue chromatographed over silica gel (5:1 hexane/ethyl acetate then 1:1 hexane/ethyl acetate 10% EtOH) to give 353 mg of a white solid (92%) which was recrystallized from methanol, mp 143-144 δ °C: PMR(300.1 MHz, $CDCl_3$) δ 8.19(s, 1H, NH), 8.00(m, 2H, aromatic), 7.73(s, 1H, H-6), 7.56(m, 1H, aromatic), 7.43(m, 2H, aromatic), 7.30(m, 5H, aromatic), 6.18(dd, J = 6.7, 3.8 Hz, 1H, H-1′), 4.56(ABq, J = 11.7, 5.4 Hz, 2H, benzyl), 4.42(dd, J = 11.3, 5.6 Hz, 1H, H-6′a), 4.30(dd, J = 11.3, 6.5 Hz, 1H, H-6′b), 4.08(dt, J = 7.8, 2.4 Hz, 1H, H-4′), 3.95(dd, J = 10.9, 2.2 Hz, H-5′a), 3.68(dd, J = 10.9, 2.6 Hz, 1H, H-5′b), 2.95(m, 1H, H-3′), 2.37(m, 1H, H-2′a), 2.26(m, 1H, H-2′b), 1.56(s, 3H, C-5 methyl); CMR(75.5 MHz) δ 167.0, 164.9, 151.2, 138.0, 136.6, 134.0, 130.2, 129.2, 129.2, 128.7, 128.3, 110.9, 85.5, 83.2, 74.0, 70.2, 64.9, 37.3, 36.8, 12.3; MS(DCI) m/e 451(M + 1), 325($M^+$ - thymine).

## Example 36

2, 3-dideoxy-3-hydroxymethyl-β-D-erythro-pentofuranosyl)thymine.

To a solution of the bis-protected nucleoside produced in Example 35 (510 mg, 1.13 mmol) in 10 mL of ethanol was added 5 mL of cyclohexene and 140 mg of Pearlman's catalyst (20% Pd on carbon) and the solution refluxed for 6 h. The catalyst was removed by filtration through a pad of Celite and the filtrate concentrated to yield the debenzylated nucleoside as a white solid (340 mg, 83%): PMR(300 MHz, $CDCl_3$) δ 8.15(br s, 1H, NH), 8.00(m, 2H, aromatic), 7.57(m, 2H, H-5 and aromatic), 7.44(m, 2H, aromatic), 6.13(t, J = 5.3 Hz, 1H, H-1′), 4.42(dd, J = 112.5, 5 Hz, 1H, H-6′a), 4.32(dd, J = 11.2, 6.3 Hz, 1H, H-6′b), 4.05(dd, J = 11.5, 2.4 Hz, 1H, H-5′a), 4.02(m, 1H, H-4′b), 3.82(dd, J = 11.5, 2.4 Hz, 1H, H-5′b), 2.90(m, 1H, H-3′), 2.34-(m, 2H, H-2′), 1.90(s, 3H, C-5 methyl). The debenzylated adduct was not purified further but was used directly. The solid was dissolved in 2 mL of methanol and stirred at ambient temperature. Meanwhile, NaH (60% in oil, 70 mg, 1.75 mmol) was washed with two 5 mL portions of pentane and 2 mL of methanol was added. This methoxide solution was added to the debenzylated nucleoside and stirred for 2 h. The solution was neutralized to a pH of 7 with acetic acid and the solution concentrated under reduced pressure. The residue was placed on a silica gel column and eluted with methylene chloride and then methylene chloride/10% methanol to give 193 mg of fully deprotected material (66% overall yield): PMR(300 MHz, $D_2O$) δ 7.71(s, 1H, H-6), 6.09(br t, J = 6.9 Hz, 1H, H-1′), 3.92(m, 1H, H-4′), 3.87(dd, J = 12.6, 2.8 Hz, 1H, H-5′a), 3.71(dd, J = 12.6, 4.8 Hz, 1H, H-5′b), 3.64(d, J = 6.1 Hz, 2H, H-6), 2.45(m, 1H, H-3′), 2.25(m, 2H, H-2′), 1.85(s, 3H, C-5 methyl).

## Example 37

1- (5-O-Benzyl-2,3-dideoxy-3[(methanesulfonyl)methyl-β-D-erythro-furanosyl)thymine.

NaH (60%, 70 mg, 1.75 mmol) was washed with pentane and 3 mL of methanol was added slowly. To this solution of NaOMe was added the bis-protected nucleoside of Example 35 (540 mg, 1.20 mmol) in 7 mL of methanol. The solution was stirred at ambient temperature for 90 min, neutralized with acetic acid, and concentrated under reduced pressure. PMR(300 MHz, $CDCl_3$) δ 8.77(br s, 1H, NH), 7.64(s, 1H, H-6), 7.30(m, 5H, aromatic), 6.10(dd, J = 6.6, 4.7 Hz, 1H, H-1′), 4.57(ABq, J = 11.8, 2.7 Hz, 2H, benzyl), 4.01(dt, J = 6.3, 3.0 Hz, 1H, H-4′), 3.83(dd, J = 10.6, 3.0 Hz, 1H, H-5′a), 3.66(m, 3H, H-6′ and H-5′b), 2.56(m, 1H, H-3′), 2.24(m, 1H, H-2′a), 2.08(m, 1H, H-2′b), 1.58(s, 3H, C-5 methyl). The residue was dissolved in 10 mL of methylene chloride and 10 mL of pyridine and cooled to 0 °C. At 0 °C was added methane sulfonyl

chloride (0.30 mL, 3.88 mmol) and stirred at 0° C for 1 h and then warned to ambient temperature for 2 h. The solution was concentrated under reduced pressure and the residue dissolved in methylene chloride and washed with saturated $NaHCO_3$. The organic fraction was dried over $MgSO_4$, concentrated, and the residue chromatographed over silica gel (ethyl acetate) to give 439 mg (86%) of a white foam which was recrystallized from methanol: mp 68-70° C; PMR(300.1 MHz, $CDCl_3$) δ; CMR(75.5 MHz) δ 165.0, 151.4, 138.1, 136.5, 129.2, 128.6, 128.3, 111.1, 85.4, 82.2, 74.0, 70.3, 69.4, 38.0, 37.7, 35.9, 12.3.

## Example 38

1-(2,3-Dideoxy-3-azidomethyl-β-D-erythro-furanosyl)thymine.

To the mesylate of Example 37 (340 mg, 0.80 mmol) was added 7 mL of ethanol, 4 mL of cyclohexene, and 90 mg of Pearlman's catalyst (20% PD on carbon). The solution was warmed to reflux for 4 h. The catalyst was removed by filtration through Celite and the filtrate concentrated under reduced pressure. The residue was dissolved in 5 ml of DMF and $NaN_3$ added (180 mg, 2.76 mmol). The solution was heated to 80° C for 2 h, cooled, filtered through glass wool and concentrated under reduced pressure. The residue was chromatographed over silica gel (ethyl acetate) to give 172 mg of a clear oil (76%): PMR(300 MHz, $D_2O$) 7.71(s, 1H, H-6), 6.16(t, J = 5.5 Hz, 1H, H-1′), 3.99(m, 1H, H-4′), 3.86(ddd, 2H, H-5′), 3.55(m, 2H, H-6′), 2.58(m, 1H, H-3′), 2.34(m, 2H, H-2′) and 1.91(s, 3H, $CH_3$). CMR(75.5 MHz, $CDCl_3$), 165.32, 151.38, 137.28, 110.97, 85.61, 84.33, 62.34, 52.74, 37.64, 36.51 and 12.56. Mass spectrum M + H = 281. Anal. Calcd. for $C_{11}H_{14}N_5O_4$; C, 46.97; H, 5.38; N, 24.90. Found C, 46.71; H, 5.57; N, 24.48.

## Example 39

$N^2$-Acetyl-9-(3-deoxy-3-[(benzyloxy)methyl]-β-D-ribopentofuranosyl)guanine.

$N^2$-Acetylguanine (966 mg; 5.0 mmoles) was persilylated by heating to 120° C under neutral atmosphere with bis-trimethylsilylacetamide (12.4 mL; 50 mmoles) for 0.5 h. The volatiles were thoroughly removed under high vacuum for 3 h. The residue and 1,2-di-O-acetyl-5-O-benzyl-3-deoxy-3-[(benzyloxy)-methyl-β-D-ribofuranose (1.45 g; 3.38 mmole) were dissolved under neutral atmosphere in dry 1,2-dichloromethane (35 ml), treated with stirring with trimethylsilyl triflate (1.5 mL; 7.76 mmole) and the mixture was heated under reflux for 1.5 h. Dichloromethane (50 mL) was added and the solution was washed with sodium bicarbonate solution and brine, dried and evaporated. The residue (2.0 g) was purified by careful chromatography on a silica gel column (50% EtOAc in hexane containing 0-25% of ethanol). $N^2$-Acetyl-7-(-2-O-acetyl-5-O-benzyl-3-deoxy-3-(benzyloxy)methyl-β-D-ribo-pentofuranosyl)guanine was eluted first and crystallized from ethanol - dichloromethane (460 mg; 24.2%). Mp 136-138° C. $H^1$ NMR 300 MHz; DMSO-$d_6$): 8.42 (s, 1H, H-8), 7.35-7.20 (m, 10 H, aromatic), 6.25 (d, $J_{1'},_{2'}$ = 2.4 Hz, 1H, H-1′), 5.59 (dd, $J_{2'},_{3'}$ = 6.4 Hz, $J_{2'},_{1'}$ = 2.4 Hz, 1H, H-2′), 4.48 (br s, 2H, benzylic $CH_2$), 4.24 (m, 1H, H-4′), 3.79 (dd, $J_{gem}$ = 11.0 Hz, $J_{5'},_{4'}$ = 2.4 Hz, 1H, H-5′), 3.64 - 3.44 (m, 3H, H-5′ + 2 x H-6′), 2.94 (m, 1H, H-3′), 2.13 (s, 3H, NHC=$OCH_3$), 1.98 (s, 3H, OC=$OCH_3$).

Mixed fractions containing the desired product and $N^2$-acetyl-9-(2-O-acetyl-5-O-benzyl-3-deoxy-3-[-(benzyloxy)methyl]-β-D-ribo-pentofuranosyl)guanine followed (250 mg) and the pure N9-isomer eluted next (360 mg; 19.0%). $H^1$ NMR (300 MHz; DMSO-$d_6$): 8.02 (br s, 1H, H-8), 7.35-7.20 (m, 10 H, aromatic), 5.90 (d, $J_{1'},_{2'}$ = 2.7 Hz, 1H, H-1′), 5.62 (dd, $J_{2'},_{3'}$ = 6.5 Hz, $J_{2'},_{1'}$ = 2.6 Hz, 1H, H-2′), 4.52 (m, 4H, benzylic $CH_2$), 4.19 (m, 1H, H-4′), 3.73 (dd, A of ABX, $J_{gem}$ = 11.0 Hz, $J_{5'},_{4'}$ = 2.6 Hz, 1H, H-5′), 3.64-3.50 (m, 3H, H-5′ + 2 x H-6′), 3.08 (m, 1H, H-3′), 2.10 (br s, 3H, NHC=$OCH_3$), 1.98 (s, 3H, OC=$OCH_3$).

## Example 40

$N^2$-Acetyl-9-(2-O-acetyl-3-deoxy-3-C-hydroxymethyl]-β-D-ribo-pentofuranosyl)guanine.

$N^2$-Acetyl-9-(2-O-acetyl-5-O-benzyl-3-deoxy-3-[(benzyloxy)methyl]-β-D-ribo-pentofuranosyl)guanine (320 mg; 0.57 mmole) was dissolved in a mixture of ethanol (3 mL) and cyclohexene (10 mL). Pearlman's catalyst (600 mg) was added under neutral atmosphere with stirring and the mixture was heated under reflux for 16 h. The mixture was filtered through Celite (washing with hot methanol), the volatiles were removed in vacuo and the residue was purified on a silica gel column (5-25%) methanol in dichloromethane) to give the desired product (100 mg; 46%). $H^1$ NMR (300 MHz, DMSO-$d_6$): 8.26 (br s, 1H, H-8), 5.94 (d, $J_{1',2'}$ = 3.7 Hz, 1H, H-1'), 5.53 (dd, $J_{2',3'}$ = 6.8 Hz, $J_{2',1'}$ = 3.7 Hz, 1H, H-2'), 5.07 (br t, J = 5.5 Hz, 1H, OH), 4.80 (br t, J = 5.5 Hz, 1H, OH), 4.05 (m, 1H, H-4'), 3.70-3.45 (m, 4H, 2 x H-5' + 2 x H-6'), 2.77 (m, 1H, H-3'), 2.14 (br s, 3H, NHC=$OCH_3$), 2.02 (s, 3H, OC=$OCH_3$).

## Example 41

9-(3-Deoxy-3-C-hydroxymethyl-β-D-ribo-pentofuranosyl)guanine.

$N^2$-Acetyl-9-(2-O-acetyl-3-deoxy-3-C-hydroxymethyl]-β-D-ribo-pentofuranosyl)guanine (90 mg; 0.236 mmole) was dissolved in methanol saturated with ammonia at 0°C (5 mL) and stirred for 14 h at rt. Formation of a precipitate was observed. The reaction mixture was triturated with water-ethanol and the product was filtered off (36 mg; 51.3%). $H^1$ NMR (300 MHz, $D_2O$): 8.01 (s, 1H, H-8), 5.94 (d, $J_{1',2'}$ = 2.1 Hz, 1H, H-1'), 4.26 (m, 1H, H-4'), 3.99 - 3.89 + 3.87 - 3.68 (m, 4H, 2 x H-5' + 2 x H-6'), 2.65 (m, 1H, H-3'), Anal. ($C_{11}H_{15}N_5O_5$ x 0.75 $H_2O$) C, H, N.

## Example 42

9-(5-O-Benzyl-3-deoxy-3-[(benzyloxy)methyl)-β-D-ribo-pentofuranosyl) guanine.

$N^2$-Acetyl-9-(2-O-acetyl-5-O-benzyl-3-deoxy3-[(benzyloxy)methyl]-β-D-ribo-pentofuranosyl)guanine was suspended in methanol saturated with ammonia at 0°C and stirred at rt for 18 h. Solvent was removed in vacuo and the residue was crystallized to give the pure product (500 mg; 58.8%). $H^1$ NMR (300 MHz, DMSO-$d_6$): 7.83 (s, 1H, H-8), 7.42-7.20 (m, 10 H, aromatic), 6.42 (br s, 2, $NH_2$), 5.68 (d, $J_{1',2'}$ = 2.1 Hz, 1H, H-1'), 4.40-4.50 (m, 5H, H-2' + benzylic), 4.14 (m, 1H, H-4'), 3.78-3.65 + 3.58-3.45 (m, 4H, 2 x H-5' + 2 x H-6'), 2.62 (m, 1H, H-1'), 2.62 (m, 1H, H-3').

## Example 43

9-(3-Deoxy-3-C-hydroxymethyl-β-D-ribo-pentofuranosyl)guanine.

9-(5-O-Benzyl-3-deoxy-3-[(benzyloxy)methyl]-β-D-ribo-pentofuranosyl)guanine (300 mg; 0.63 mmole) was combined under neutral atmosphere with pearlman's catalyst (2.0 g) Ethanol, (5 mL) and cyclohexene (15 mL) were added and the mixture was heated under reflux with stirring for 14 h. The mixture was filtered through Celite, concentrated in vacuo and then through a short $C_{18}$ column. Volatiles were removed in vacuo and the residue was purified by $C_{18}$ HPLC. Crystallization from water-methanol gave the product (67 mg; 35.8%), identical to the material obtained from Example 41.

## Example 44

9-(5-O-Benzyl-2,3-dideoxy-3-[(benzyloxy)methyl-β-D-ribo-pentofuranosyl)guanine.

9-(5-O-Benzyl-3-deoxy-3-[(benzyloxy)methyl)-β-D-ribo-pentofuranosyl)guanine (500 mg; 1.05 mmole) was dissolved in dry 1,2-dichloromethane (25 mL) and treated under neutral atmosphere with N,N-thiocarbonyl-diimidazole (214.6 mg; 1.2 mmole). The mixture was heated under reflux with stirring for 1 h and the volatiles were removed in vacuo. Xylene (20 mL) was added and the evaporation was repeated followed by 2 h under high vacuum to give crude 9-(2-O-imidazolothio-carbonyl-5-O-benzyl-3-deoxy-3-[-(benzyloxy)methyl-β-D-ribo-pentofuranosyl)guanine, which was used without purification for the next step. $H^1$NMR (300 MHz; DMSO-$d_6$): 8.49 (s, 1H, H-8), 6.55 (br s, 2H, $NH_2$), 6.26 (dd, $J_{2',3'}$ = 6.3 Hz, $J_{2',1'}$ = 1.2 Hz, 1H, H-2'), 6.08 (d, $J_{1',2'}$ = 1.5 Hz, 1H, H-1'), 3.44 (m, 1H, H-3'). Crude product was dissolved under neutral atmosphere in dry 1,4-dioxane (15 mL) containing AIBN (45 mg) and tri-n-butyltin hydride (1.0 mL; 3.71 mmole) was added with stirring. The mixture was heated under reflux for 12 h and the volatiles were removed in vacuo. Column chromatography of the residue (2-10% methanol in dichloromethane) gave the desired product (220 mg; 45.5% overall yield). $H^1$ NMR (300 MHz; DMSO-$d_6$): 7.85 (br s, 1H, H-8), 7.4-7.2 (m, 10 H, aromatic), 6.42 (br s, 2H, $NH_2$), 5.99 (dd, $J_{1',2A'}$ = 7.5 Hz, $J_{1',2B'}$ = 3.6 Hz, 1H, H-1'), 4.48 (narrow AB, 2H, benzylic), 4.44 (narrow AB, 2H, benzylic), 3.98 (m, 1H, H-4'), 3.67 (dd, AB of ABX, 1H, H-5'), 3.55-3.45 (m, 3H, H-5' + 2 x H-6'), 2.7 (m, 1H, H-3'), 2.5-2.36 (m, 1H, H-2'), 2.30-2.20 (m, 1H, H-2').

## Example 45

9-(2,3-Dideoxy-3-C-hydroxymethyl-β-D-ribo-pentofuranosyl)guanine.

9- (5-O-Benzyl-2,3-dideoxy-3-[(benzyloxy)methyl-β-D-ribo-pentofuranosyl)guanine (200 mg; 0.433 mmole) was combined under neutral atmosphere with Pearlman's catalyst (600 mg). Ethanol (2 mL) and cyclohexene (10 mL) were added and the mixture was heated under reflux with stirring for 14 h. The mixture was filtered through Celite (washing with hot methanol) and the volatiles were removed in vacuo. The residue (85 mg) was purified by $C_{18}$ HPLC and the main fractions were recrystallized from water-methanol-ethanol to give the desired product (51 mg; 41.9%). $H^1$ NMR (300 MHz; $D_2O$): 8.01 (s, 1H, H-8), 6.20 (dd, $J_{1',2A'}$ = 7.5 Hz, $J_{1',2B'}$ = 3.4 Hz, 1H, H-1'), 4.07 (m, 1H, H-4'), 3.83 (dd, A of ABX, $J_{gem}$ = 12.6 Hz, $J_{5A',4'}$ = 3.0 Hz, 1H, H-5A'), 3.65 (B of ABX, $J_{gem}$ = 12.7 Hz, $J_{5B',4'}$ = 5.3 Hz, 1H, H-5B'), 3.73 (d, $J_{6A',3'}$ = $J_{6B',3'}$ = 5.4 Hz, 2H, 2 x H-6'), 2.7-2.6 (m, 2H, H-3' + H-2'), 2.48-2.38 (m, 1H, H-2'). Anal. ($C_{11}H_{15}N_5O_4$ x 0.2 $H_2O$) C, H, N.

## Example 46

1-(2-O-Acetyl-5-O-benzyl-3-deoxy-3-[(benzoyloxy)methyl)-β-D-ribofuranosyl)uracil.

Tin chloride (1.45 mL, 12.4 mmol) was added to a solution of the acetate from Example 1 (3.7 g, 8.23 mmol), $CH_3CN$ (180 mL), uracil (1.16 g, 10.3 mmol), hexamethyldisilazane (1.94 mL, 9.20 mmol) , and chlorotrimethyl silane (1.25 mL, 9.85 mmol). The solution was stirred at ambient temperature for 4 h and then diluted with $CHCl_3$. The mixture was then washed with saturated $NaHCO_3$ and brine, and the organic fraction dried ($MgSO_4$) and concentrated in vacuo. The residue was purified by flash column chromatography on a silica gel column eluted with 3% MeOH -97% $CH_2Cl_2$ to leave a white solid (3.2 g, 79%) mp 46-47° C; $^1H$ NMR (300.1 MHz, $CDCl_3$) δ 8.93 (s, 1H, NH), 7.98 (m, 2H, aromatic), 7.87 (d, J = 8.2 Hz, 1H, H-6), 7.56 (m, 1H, aromatic), 7.43 (m, 2H, aromatic), 7.29 (m, 5H, aromatic) 5.97 (d, J = 2.3 Hz, 1H, H-1'), 5.54 (dd, J = 6.1, 2.3 Hz, 1H, H-2'), 5.32 (dd, J = 8.1, 1.8 Hz, 1H, H-5), 4.51 (s, 2H, benzyl), 4.46 (m, 2H, H-4' and H-6'), 3.98 (dd, J = 11.0, 1.9 Hz, 1H, H-5'a), 3.68 (dd, J = 11.0, 2.3 Hz, 1H, H-5'b), 3.04 (m, 1H, H-3'), 2.08 (s, 3H, acetate); $^{13}C$ NMR (75.5 MHz, $CDCl_3$) δ 169.3, 166.1, 163.1, 150.0, 140.0, 137.0, 133.4, 129.6, 129.4, 128.7, 128.5, 128.4, 102.1, 89.4, 82.0, 76.8, 73.8, 69.3, 60.5, 39.7, 20.7; IR(KBr) 1750, 1718, 1690 $cm^{-1}$; MS (DCI) m/e 495($M^+$1), 435($M^+$-OAc), 383 ($M^+$-uracil).

## Example 47

1-(5-O-Benzyl-3-deoxy-3-[(benzoyloxy)methyl) -2-hydroxy-β-D-ribofuranosyl)uracil.

A solution of the acetate from Example 46 (0.260 g, 0.526 mmol) in methanol (4 mL) was cooled to 0° C and then saturated with ammonia. The mixture was stirred for 1 h at 0° C and 1.5 h at 20° C. The solvent was then removed in vacuo and the residue was purified by flash chromatography on silica gel with 5% MeOH - 95% $CH_2Cl_2$ as eluent to leave a white solid (0.173 g, 73%); mp 67-70° C; $^1H$ NMR (300.1 MHz, ($CDCl_3$) δ 10.05 (s, 1H, NH), 8.17 (d, J = 8.1 Hz, 1H, H-6), 7.99 (m, 2H, aromatic), 7.55 (m, 1H, aromatic), 7.41 (m, 2H, aromatic), 7.26 (m, 5H, aromatic), 5.77 (s, 1H, H-1′), 5.19 (dd, J = 8.1, 2.1 Hz, 1H, H-5), 4.92 (d, J = 2.6 Hz, 1H, OH), 4.71 (dd, J = 11.3, 6.8 Hz, 1H, H-6′a), 4.43 (m, 5H, H-2′, H-4′, H-6′b, benzyl), 4.07 (d, J = 9.6 Hz, 1H, H-5′a), 3.73 (d, J = 9.5 Hz, 1H, H-5′b), 2.73 (m, 1H, H-3′); $^{13}C$ NMR (75.5 MHz, $CDCl_3$) δ 167.0, 164.9, 151.6, 141.3, 137.8, 133.8, 130.3, 130.2, 129.1, 129.0, 128.6, 128.5, 101.9, 93.3, 83.6, 77.3, 74.1, 69.1, 61.4, 39.6; IR(film) 3398, 3212, 1700 $cm^{-1}$; MS(DCI) m/e 453 ($M^+$1), 435($M^+$-OH),) 341($M^+$-uracil).

## Example 48

1-(5-O-Benzyl-3[(benzoyloxy)methyl)-2,3-dideoxy-β-D-erythro-furanosyl)uracil.

Thiocarbonyl diimidazole (1.8 g, 10.0 mmol) was added to a solution of the alcohol from Example 47 (1.82 g, 4.02 mmol) in THF (45 mL). The mixture was warmed to reflux for 7 hours, cooled, and the solvent removed in vacuo. The residue was purified by flash chromatography over silica gel with 3% MeOH - 97% $CH_2Cl_2$ as eluent to leave a white solid (1.635 g, 72%); $^1H$ NMR (300.1 MHz, $CDCl_3$), δ 9.10 (s, 1H, NH), 7.96 (m, 2H, aromatic), 7.78 (d, J = 8.2 Hz, 1H, H-6), 7.52 (m, 1H, aromatic), 7.38 (m, 2H, aromatic), 7.27 (m, 6H, aromatic), 6.02 (d, J = 2.5 Hz, 1H, imidazole), 5.98 (dd, J = 6.0, 2.5 Hz, 1H, H-1′), 5.30 (dd, J = 8.1, 1.9 Hz, 1H, H-5), 4.48 (m, 3H, benzyl, H-6′a), 4.34 (m, 2H, H-4′, H-6′b), 3.95 (m, 3H, H-2′, H-5′a), 3.66 (dd, J = 10.9, 2.3 Hz, H-5′b), 3.15 (m, 1H, H-3′); $^{13}C$ NMR (75.5 MHz, $CDCl_3$) δ 195.1, 166.1, 163.2, 150.0, 140.4, 137.1, 133.4, 129.7, 129.4, 128.7, 128.5, 128.3, 128.0, 102.3, 89.3, 84.7, 82.0, 73.8, 69.6, 60.5, 60.3, 40.1; IR(KBr) 1720, 1690 $cm^{-1}$.

A solution of the benzoate prepared above, dioxane (70 mL), bis (tributyltin)oxide (7 mL), and polymethylhydrosiloxane (7 mL) was treated with AIBN (0.032 g, 0.195 mmol). The mixture was warmed to 100° C for 2 h, cooled, and the mixture was then passed through Celite. The filtrate was concentrated and the residue was purified by flash chromatography on a silica gel column with 3% MeOH - 97% $CH_2Cl_2$ as eluent to leave a white solid (1.15 g, 94%); $^1H$ NMR (300.1 MHz, $CDCl_3$) δ 9.23 (s, 1H, NH) , 7.98 (m, 3H, H-6, aromatic), 7.56 (m, 1H, aromatic), 7.42 (m, 2H, aromatic), 7.30 (m, 5H, aromatic), 6.14 (dd, J = 6.6, 3.1 Hz, 1H, H-1′), 5.31 (dd, J = 8.2, 1.8 Hz, 1H, H-5), 4.51 (s, 2H, benzyl), 4.40 (dd, J = 11.4, 5.6 Hz, 1H, H-6′a), 4.28 (dd, J = 11.4, 6.4 Hz, 1H, H-6′b), 4.08 (d, J = 8.2 Hz, 1H, H-4′), 3.94 (dd, J = 10.8, 2.1 Hz, 1H, H-5′a), 3.70 (dd, J = 10.8, 2.4 Hz, 1H, H-5′b), 2.89 (m, 1H, H-3′), 2.29 (m, 2H, H-2′); $^{13}C$ NMR (75.5 MHz, $CDCl_3$) δ 167.0, 164.3, 151.1, 141.1, 137.9, 134.0, 130.2, 129.3, 129.2, 128.8, 128.5, 102.0, 86.0, 83.5, 74.2, 70.0, 64.7, 37.0, 36.9; IR (film) 1710, 1684: MS (DCI) m/e (437($M^+$-1), 325 ($M^+$-uracil).

## Example 49

1-(5-O-Benzyl-2,3-dideoxy-3[(methanesulfonyl)methyl)-β-D-erythro-furanosyl)uracil.

A mixture of the benzoate from Example 48 (1.23 g, 2.82 mmol) in MeOH (6 mL) was added to a solution of sodium hydride (0.101 g, 4.20 mmol) in MeOH (6 mL). The mixture was stirred at ambient temperature for one h, neutralized with acetic acid and then concentrated in vacuo. The residue was dissolved in $CH_2Cl_2$ (25 mL) and pyridine (25 mL). The solution was cooled to 0° C, methanesulfonyl

chloride (0.57 mL, 7.36 mmol) was added and the mixture was stirred at 0° C for 1 h.

The ice bath was removed and the solution was stirred for 3 h at ambient temperature and then concentrated. The residue was dissolved in $CH_2Cl_2$ and then washed with saturated $NaHCO_3$. The aqueous layer was reextracted with $CH_2Cl_2$, the combined organic layers were dried and concentrated. The residue was purified by flash chromatography over silica gel and eluted with 3% MeOH - 97% $CH_2Cl_2$ to leave a white solid (0.724 g, 63%); mp 56-59° C; $^1H$ NMR (300.1 MHz, $CDCl_3$) δ 8.94 (s, 1H, NH), 7.89 (d, J = 8.1 Hz, 1H, H-6), 7.30 (m, 5H, aromatic), 6.06 (dd, J = 6.5, 3.9 Hz, 1H, H-1′), 5.31 (d, J = 8.1 Hz, 1H, H-5), 4.52 (s, 2H, benzyl), 4.20 (m, 2H, H-6′), 4.04 (d, J = 7.6 Hz, 1H, H-4′), 3.89 (dd, J = 10.8, 2.3 Hz, 1H, H-5′a), 3.64 (dd, J = 10.8, 2.3 Hz, 1H, H-5′b), 2.97 (s, 3H, methyl), 2.81 (m, 1H, H-3), 2.23 (m, 2H, H-2); $^{13}C$ NMR (75.5 Hz, $CDCl_3$) δ 163.3, 150.2, 140.3, 137.1, 128.6, 128.3, 128.0, 101.6, 85.3, 82.1, 73.8, 69.6, 68.4, 37.5, 37.3, 36.0; IR(film) 1686, 1354 $cm^{-1}$; MS(DCI) m/e 411($M^+$1), 299($M^+$-uracil); Anal. Calcd for $C_{18}H_{22}N_2O_7S$; C,52.67; H,5.40; N,6.85. Found: C,52.60; H,5.31; N, 6.54.

## Example 50

1(2,3-Dideoxy-3-[(methanesulfonyl)methyl]-β-D-erythro-furanosyl)uracil

Pearlman's catalyst (0.40 g) was added to a heterogeneous mixture of the mesylate from Example 49 (0.660 g, 1.61 mmol), cyclohexene (8 mL), and ethanol (14 mL). The mixture was warmed to reflux for 36 h. The mixture was then cooled, filtered through Celite and concentrated under reduced pressure. The residue was purified by flash chromatography on a silica gel column with 7% MeOH - 93% $CH_2Cl_2$ as eluent to give a white foam (0.348 g, 67%); mp 107-109° C; $^1H$ NMR (300.1 MHz, DMSO-$d_6$) δ 11.30 (s, 1H, NH), 7.97 (d, J = 8.1 Hz, 1H, H-6), 6.02 (dd, J = 6.8, 4.5 Hz, 1H, H-1′), 5.61 (dd, J = 8.1, 1.7 Hz, 1H, H-5), 5.14 (t, J = 5.3 Hz, 1H, OH), 4.28 (d, J = 6.2 Hz, 2H, H-6′), 3.84 (m, 1H, H-4′), 3.71 (m, 1H, H-5′a), 3.70 (m, 1H, H-5′b), 3.22 (s, 3H, methyl), 2.66 (m, 1H, H-3′), 2.19 (m, 2H, H-2); $^{13}C$ NMR (75.5 MHz, DMSO-$d_6$) δ 163.3, 150.5, 140.7, 101.4 84.4, 82.9, 70.4, 61.4, 37.2, 36.6, 34.9; IR(film) 1684, 1348 $cm^{-1}$; MS(DCI) m/e 321-($M^+$1), 209($M^+$-uracil).

## Example 51

1-(2,3-Dideoxy-3-azidomethyl-β-erythro-furanosyl)uracil.

Sodium azide (0.21 g, 3.23 mmol) was added to a solution of the alcohol from Example 50 (0.298 g, 0.924 mmol) in DMF (6 mL) and the mixture was warned to 80° C for 30 minutes. The solution was cooled and then poured onto water (50 mL) and extracted with $CH_2Cl_2$ and ethyl acetate. The combined organic fractions were dried and concentrated. The residue was purified by flash chromatography over silica gel (7% MeOH - 93% $CH_2Cl_2$) to leave a white solid (0.199 g, 81%); mp 135-137° C; $^1H$ NMR (300.1 MHz, DMSO) δ 11.25 (s, 1H, NH), 7.94 (d, J = 8.1 Hz, 1H, H-6), 5.97 (dd, J = 6.3, 4.6 Hz, 1H, H-1′), 5.57 (d, J = 8.1 Hz, 1H, H-5), 5.09 (s, 1H, OH), 3.70 (m, 2H, H-5′a, H-4′), 3.52 (m, 1H, H-5′b), 3.48 (d, J = 6.4 Hz, 2H, H-6′), 2.47 (m, 1H, H-3′), 2.13 (m, 2H, H-2′); $^{13}C$ NMR (75.5 MHz, DMSO-$d_6$) δ 163.3, 150.5, 140.7, 101.3, 84.3, 83.8, 61.2, 52.0, 37.4, 36.0; IR(film) 3448, 2102, 1686 $cm^{-1}$; MS(DCI) m/e 268($M^+$1), 240 ($M^+$-$N_2$), 156($M^+$-uracil).

## Example 52

1,2:5,6-Di-O-isopropylidene-3-deoxy-3-C-fluoromethyl-α-D-gluco-hexofuranose.

1,2:5,6-Di-O-isopropylidene-3-C-hydroxymethyl-α-D-gluco-hexofuranose (6.0 g; 21.87 mmole) was dissolved in dry THF (60 mL) and treated under neutral atmosphere (stirring) with DAST (17.63 g, 14.4 mL; 109.36

mmole). Stirring was continued for 14 h and the mixture was poured carefully into a vigorously stirred bicarbonate solution saturated with sodium chloride. The resulting suspension was extracted with dichloroethane, rhe organic layer was washed with brine, dried and evaporated. The residue was purified on a silica gel column (10-60% EtOAc in hexane) to give the product (2.4 g; 39.7%). $H^1$ NMR (300 MHz; ($CDCl_3$): 5.78 (d, $J_{1,2}$ = 3.9 Hz, 1H, H-1), 4.83 (br t, J = 3.6 Hz, 1H, H-2), 4.85 - 4.65 (m, 2H, H-7), 2.34 (m, 1H, H-3), 1.49 + 1.34 + 1.31 + 1.29 (4 x s, 4 x 3H, acetonide $CH_3$).

## Example 53

1,2-O-Isopropylidene-5-O-benzyl-3-deoxy-3-C-fluoromethyl-α-ribo-pentofuranose.

1.2:5,6-Di-O-isopropylidene-3-deoxy-3-C-fluoromethyl-α-D-gluco-hexofuranose (5.5 g; 19.9 mmole) was dissolved in a mixture of methanol (75 mL) and water (25 mL) with stirring and 0.2 N sulfuric acid was added (50 L). Stirring was continued for 20 h. The mixture was concentrated in vacuo to one-half or the original volume. The pH was adjusted to neutral with sodium bicarbonate and the volatiles were removed under high vacuum. The residue was taken up in dichloromethane-ethyl acetate (1:1), dried, filtered, and evaporated to give 1,2-O-isopropylidene-3-C-fluoromethyl-α-D-ribo-hexofuranose (4.7 g) , which was used directly for the next step. $H^1$ NMR (300 MHz; $CDCl_3$): 5.79 (d, $J_{1,2}$ = 3.6 Hz, 1H, H-1), 4.74 br t, 1H, H-2), 4.71 (dd, $J_{7,F}$ = 46.5 Hz, $J_{7,3}$ = 7.5 Hz, 2H, 2 x H-7), 3.88 (m, 1H, H-4), 3.68 (m, 3H, H-5 + 2 x H-6), 2.83 (br s, 1H, OH), 2.50-2.34 (m, 2H, H-3 + OH), 1.48 + 1.29 (2 x s, 2 x 3H, acetonide $CH_3$). This material was dissolved in a mixture of ethanol (50 mL) and cater (25 mL) with vigorous stirring. The cloudy solution was treated with a saturated sodium bicarbonate solution (7.5 mL) and a suspension of sodium metaperiodate (5.16 g; 24.13 mmole) in water (25 mL). Stirring at rt was continued for 3 h. Ethylene glycol (1 mL) was added and after 25 minutes, the mixture was treated with sodium borohydride (454 mg; 12 mmoles). After 1 h, acetone (2 mL) was added and after further 0.5 h the mixture was concentrated in vacuo to one-half of its original volume. Extraction with EtOAc, drying and evaporation gave crude 1,2-O-isopropylidene-3-C-fluoromethyl-α-D-ribo-pentofuranose, used directly for the next step. $H^1$ NMR (300 MHz; $CDCl_3$): 5.82 (d, $J_{1,2}$ = 3.6 Hz, 1H, H-1), 4.70 (br t, J = 4.2 Hz, 1H, H-2), 4.75 (ddd, $J_{6A,F}$ = 46.2, $J_{gem}$ = 9.3 Hz, $J_{6A,3}$ = 6.9 Hz, 1H, H-6A), 4.50 (ddd, $J_{6B,F}$ = 47.5 Hz, $J_{gem}$ = 9.3 Hz, $J_{6B,3}$ = 7.5 Hz, 1H, H-6B), 4.05 (dm, J = 10.2 Hz, 1H, H-4), 3.87 (m, 1H, H-5A), 3.58 (ddd, $J_{gem}$ = 12.0 Hz, J = 7.5 Hz, J = 3.9 Hz, 1H, H-5B), 2.49 (m, 1H, H-3), 1.96 (br t, 1H, OH), 1.47 + 1.29 (2 x s, 2 x 3H, acetonide $CH_3$). This material was dissolved in dry toluene (30 mL) and the volatiles were removed in vacuo. The residue was dissolved in dry DME (15 mL) under neutral atmosphere (stirring) and treated with sodium hydride (60% suspension in oil, 680 mg; 17 mmoles). After 0.5 h, benzyl bromide (3.24 g, 2.25 mL; 18.91 mmole) was added and stirring was continued for 2.5 h. The volatiles were removed in vacuo. The residue was taken up in diethyl ether, washed with brine, dried and evaporated. The residue was purified on a silica gel column (12.5 - 25% EtOAc in hexane) to give the title product (3.2 g; 54% over four steps). $H^1$ NMR (300 MHz; $CDCl_3$): 7.37-7.20 (m, 5H, aromatic), 5.84 (d, $J_{1,2}$ = 3.6 Hz, 1H, H-1), 4.70 (t, J = 3.9 Hz, 1H, H-2), 4.81-4.38 (m, 2H, 2 x H-6), 4.55 (AB, 2H, benzylic $CH_2$), 4.08 (m, 1H, H-4), 3.69 (A of ABX, $J_{gem}$ = 10.5 Hz, $J_{5A,4}$ = 3.0 Hz, 1H, H-5A), 3.53 (B of ABX, $J_{gem}$ = 10.8 Hz, $J_{5,4}$ = 4.2 Hz, 1H, H-5B), 2.48 (m, 1H, H-3), 1.47 + 1.29 (2 x s, 2 x 3H, acetonide $CH_3$).

## Example 54

1,2-O-Isopropylidene-5-O-benzyl-3-deoxy-3-C-fluoromethyl-α-D-ribo-pentofuranose.

1,2-O-Isopropylidene-5-O-benzyl-3-deoxy-3-C-hydroxymethyl-α-D-ribo-pentofuranose (800 mg; 2.68 mmole) was dissolved in dry THF (5 ml) under neutral atmosphere (stirring) and treated at 5 °C with dimethylaminosulfur trifluoride (1.5 mL; 15.37 mmole). The reaction mixture was allowed to reach rt and stirring was continued for 14 h. The reaction mixture was then poured carefully with vigorous stirring into a cold bicarbonate solution and the resulting suspension was extracted with dichloromethane. Drying and evaporation yielded the crude material (940 mg) which was purified subsequently on a silica gel column (dichloromethane to 25% EtOAc in dichloromethane) to give product (514 mg; 64.7%) identical to the one

obtained from Example 53.

## Example 55

1,2-Di-O-acetyl-5-O-benzyl-3-deoxy-3-C-fluoromethyl-β-D-ribo-pentofuranose.

1,2-O-Isopropylidene-5-O-benzyl-3-deoxy-3-C-fluoromethyl-α-D-ribo-pentofuranose (1.05 g; 3.543 mmole) was treated with 80% acetic acid (15 mL) at 100-110 °C for 2.5 h. The volatiles were removed in vacuo. Dry toluene (25 mL) was added and removed in vacuo. The residue was dissolved in dry pyridine (6 mL) and treated with acetic anhydride (3 mL) for 14 h. Evaporation gave the crude material which was purified by filtration through a silica gel column (10-25% EtOAc in hexane) to provide product (0.99 g; 82.1%) containing 30% of the α-D isomer. $H^1$ NMR (300 MHz; $CDCl_3$): 7.33-7.20 (m, 5H, aromatic), 6.39 (dd, $J_{1,2}$ = 4.2 Hz, J = 1.5 Hz, 0.3 H, H-1 α-isomer), 6.07 (s, 0.7 H, H-1), 5.34 (dd, $J_{2,1}$ = 4.2 Hz, J = 9.0 Hz, 0.3 H, H-2 α-isomer), 5.26 (d, J = 5.1 Hz, 0.7 H, H-2), 4.70-4.43 (m, 2H, 2 x H-6), 4.55-4.51 (m, 2H, benzylic $CH_2$), 4.34 (m, 0.3 H, H-4 α-isomer), 4.23 (dt, J = 9.3 Hz, J = 4.5 Hz, 0.7 H, H-4), 3.62-3.47 (m, 2H, 2 x H-5), 2.87 (m, 1H, H-3), 2.07 + 1.91 (2 x s, 2 x 2.1H, 2 x $OC=OCH_3$), 2.04 + 2.03 (2 x s, 2 x 0.9 H, 2 x $OC=OCH_3$ α-isomer).

## Example 56

1-(2-O-Acetyl-5-O-benzyl-3-deoxy-3-C-fluoromethyl-ribo-pentofuranosyl)thymine.

1,2-Di-O-acetyl-5-O-benzyl-3-deoxy-3-C-fluoromethyl-β-D-ribo-pentofuranose (0.95 g; 2.79 mmole), dry thymine (414 mg; 3.28 mmole) and dry acetonitrile (35 mL) were placed in a reaction vessel with stirring (neutral atmosphere) and treated at rt with HMDS (583 mg, 604 µL; 3.61 mmol) chlorotrimethylsilane (428 mg, 500 µL; 3.94 mmol) and trifluoromethanesulfonic acid (590 mg, 349 µL). The temperature was raised to 50-55 °C (bath) and after 3 h at this temperature, the reaction mixture was cooled down and the volatiles were removed in vacuo. The residue was taken up in dichloromethane (100 mL), washed with sodium bicarbonate solution, brine, dried and evaporated to give (976 mg; 86.1%). This material was sufficiently pure for the next step. $H^1$ NMR (300 MHz; $CDCl_3$): 7.96 (br s, 1H, NH), 7.57 (br s, 1H, H-6), 7.38 - 7.25 (m, 5H, aromatic), 5.95 (d, $J_{1',2'}$ = 3.6 Hz, 1H, H-1'), 5.44 (dd, $J_{2',3'}$ = 6.9 Hz, $J_{2',1'}$ = 3.6 Hz, 1H, H-2'), 4.69-4.40 (m, 2H, $CH_2F$), 4.60 (br s, 2H, benzylic $CH_2$), 4.33 (dt, J = 7.5 Hz, $J_{4',5A'}$ = 2.1 Hz, 1H, H-4'), 3.93 (dd, $J_{gem}$ = 10.8 Hz, $J_{5',4'}$ = 2.1 Hz, 1H, H-5A'), 3,60 (dd, $J_{gem}$ = 10.8 Hz, $J_{5B',4'}$ = 2.4 Hz, 1H, H-5B'), 2.95 (dm, $J_{3',F}$ = 21.6 Hz, 1H, H-3'), 2.09 (s, 3H, $OC=OCH_3$), 1.56 (br s, 3H, $CH_3$).

## Example 57

1-(5-O-Benzyl-3-deoxy-3-C-fluoromethyl-β-D-ribo-pentofuranosyl)thymine.

1-(2-O-Acetyl-5-O-benzyl-3-deoxy-3-C-fluoromethyl-ribo-pentofuranosyl)thymine (900 mg; 2.21 mmol) was dissolved in methanol saturated with ammonia (25 mL) and the solution was stirred at rt for 14 h. The volatiles were removed in vacuo and the residue was crystallized from diethyl ether to give analytically pure product (585 mg; 72.6%). $H^1$ NMR (300 MHz; $CDCl_3$): 8.00 (br s, 1H, NH), 7.35-7.25 (m, 5H, aromatic), 5.75 (br s, 1H, H-1'), 5.5 (m, 1H, OH), 4.84 (ddd, $J_{6A',F}$ = $J_{6B',F}$ = 46.2 Hz, $J_{gem}$ = 9.6 Hz, $J_{6A',3'}$ = $J_{6B',3'}$ = 5.7 Hz, 2H, 2 x H-6'), 4.59 (bs, 2H, benzylic $CH_2$) - 4.06 (A of AB, $J_{gem}$ = 11.1 Hz, 1H, H-5'), 3.64 (B of AB, $J_{gem}$ = 10.5 Hz, 1H, H-5'), 2.75 (m, 1H, H-3'), 1.42 (br s, 1H, $CH_3$). Anal. ($C_{18}H_{21}F_1N_2O_5$) C, H, N.

## Example 58

1-(5-O-Benzyl-2,3-dideoxy-3-C-fluoromethyl-β-D-erythro-pentofuranosyl)thymine.

1-(5-O-Benzyl-3-deoxy-3-C-fluoromethyl-β-D-ribo-pentofuranosyl)thymine (525 mg; 1.44 mmol) was dissolved in dry acetonitrile (5 mL) under neutral atmosphere (stirring) and treated with DMAP (506 mg; 4.14 mmol) and phenyl chlorothionoformate (596 mg; 3.45 mmol). After 14 h at rt, the solvent was removed in a stream of nitrogen and the residue was purified on a silica gel column (10-75% EtOAc in hexane) to give 1-(2-O-phenoxy-thiocarbonyl-5-O-benzyl-3-deoxy-3-C-fluoromethyl-β-D-ribo-pentofuranosyl)thymine (255 mg; 35.4%). $H^1$ (300 MHz; $CDCl_3$): 9.18 (br s, 1H, NH), 7.50 (br s, 1H, H-6), 7.40-7.22 (m, 8 H, aromatic), 7.09 (m, 2H, aromatic), 6.11 (d, $J_{1',2'}$ = 3.6 Hz, 1H, H-1'), 6.00 (dd, J = 6.9 Hz, $J_{2',1'}$ = 3.6 Hz, 1H, H-2') 4.8-4.5 (m, 2H, $CH_2F$), 4.60 (s, 2H, benzylic $CH_2$), 4.41 (dt, J = 7.2 Hz, $J_{4',5A'}$ = $J_{4',5B'}$ = 2.4 Hz, 1H, H-4', 3.93 (dd, $J_{gem}$ = 10.7 Hz, $J_{5A',4'}$ = 2.4 Hz, 1H, H-5A'), 3.64 (dd, $J_{gem}$ = 10.8 Hz, $J_{5B',4'}$ = 2.7 Hz, 1H, H-5B'), 3.21 (dm, $J_{3',F}$ = 21.3 Hz, 1H, H-3'), 1.54 (br s, 3H, $CH_3$). This material was dissolved in dry toluene containing AIBN (25 mg) and treated under neutral atmosphere (stirring) with tri-n-butyltin hydride at 80-90° (bath) for 1 h. The volatiles were removed in vacuo and the residue was purified on a silica gel column to give the title product, which was crystallized from ethanol-diethyl ether. Yield: 122 mg (77.9%). Mp 111-112° C. $H^1$ NMR (300 MHz; $CDCl_3$): 8.83 (br s, 1H, NH), 7.70 (br s, 1H, H-6), 7.35-7.25 (m, 5H, aromatic), 6.12 (dd, $J_{1',2A'}$ = 6.9 Hz, $J_{1',2B'}$ = 4.5 Hz, 1H, H-1'), 4.59 (AB, 2H, benzylic $CH_2$), 4.6-4.45 (m, 1H, H-6A'), 4.42-4.28 (m, 1H, H-6B'), 4.07 (dt, J = 7.5 Hz, J = 2.4 Hz, 1H, H-4'), 3.91 (dd, $J_{gem}$ = 10.8 Hz, $J_{5'A,4'}$ = 2.4 Hz, 1H, 5'A), 3.62 (dd, $J_{gem}$ = 10.8 Hz, $J_{5'B,4'}$ = 2.7 Hz, 1H, H-5'B), 2.80 (m, 1H, H-3'), 2.27 (ddd, $J_{gem}$ = 13.8 Hz, J = 8.7 Hz, $J_{2A',1'}$ = 6.9 Hz, 1H, H-2A'), 2.13 (ddd, $J_{gem}$ = 13.8 Hz, J = 8.4 Hz, $J_{2'B,1'}$ = 4.5 Hz, 1H, H-2'B), 1.57 (d, $J_{allylic}$ = 1.2 Hz, 3H, $CH_3$). Anal. ($C_{18}H_{21}F_1N_2O_4$) C, H. N.

## Example 59

1-(5-O-Benzyl-2,3-dideoxy-3-C-hydroxymethyl-β-D-erythro-furanosyl)thymine.

1- (5-O-Benzyl-2,3-dideoxy-3-[benzyloxy(methyl)]-β-D-erythro-furanosyl)thymine (240 mg; 0.533 mmole) was dissolved in methanol, saturated with ammonia at 0° C (15 mL) and stirred at rt for 72 h. Removal of volatiles in vacuo and chromatography of the residue on a silica gel column (20-60% EtOAc in $CH_2Cl_2$ then 5% EtOH in ETOAC) yielded crude product. This material was crystallized from $Et_2O$ -$CHCl_3$ - EtOAc. Yield: 126 mg (68.2%). Mp 114-115° C. $H^1$ NMR (300 MHz; $CDCl_3$): 7.61 (br s, 1H, H-6), 7.34-7.25 (m, 5H, aromatic), 5.99 (dd, $J_{1',2'A}$ = 6.9 Hz, $J_{1',2'B}$ = 4.6 Hz, 1H, H-1'), 4.79 (t, J = 5.1 Hz, 1H, OH), 4.54 (s, 2H, benzylic $CH_2$), 3.92 (m, 1H, H-4'), 3.77 (dd, $J_{gem}$ = 10.7 Hz, $J_{5'A,4}$ = 2.5 Hz, 1H, H-5'A), 3.61 (dd, $J_{gem}$ = 10.8 Hz, $J_{5'B,4'}$ = 4.1 Hz, 1H, H-5'B), 3.44 (t, $J_{6'A,3'}$ = $J_{6'B,4'}$ = 5.3 Hz, 2H, $CH_2OH$), 2.41 (m, 1H, H-3'), 2.19 - 2.08 (m, 1H, H-2'), 2.05 - 1.96 (m, 1H, H-2'), 1.50 (d, $J_{allylic}$ = 0.9 Hz, 3H, $CH_3$). Anal. ($C_{18}H_{22}N_2O_5$) C, H, N.

## Example 60

1-(5-O-Benzyl-2,3-dideoxy-3-C-fluoromethyl-β-D-erythro-furanosyl)thymine.

1-(5-O-Benzyl-2,3-dideoxy-3-C-fluoromethyl-β-D-erythro-furanosyl)thymine (105 mg; 0.303 mmole) was dissolved in a mixture of dry THF (2 mL) and dichloromethane (1 mL) and treated at rt with stirring (argon) with dimethylaminosulfur trifluoride (0.4 mL; 4.1 mmole). After 16 h at rt, the reaction mixture was poured with vigorous stirring into a cold sodium bicarbonate solution. Extraction with dichloromethane gave the crude material (90 mg), which was purified on a silica gel column (20-50% EtOAc in dichloromethane). Yield: 61 mg (57.8%). This material is identical to product obtained in Example 58.

## Example 61

1-(2,3-Dideoxy-3-C-fluoromethyl-β-D-erythro-pentofuranosyl)thymine.

1-(2,3-Dideoxy-3-C-fluoromethyl-β-D-erythro-furanosyl)thymine (110 mg; 0.316 mmole) was suspended in a mixture of ethanol (1 mL) and cyclohexene (3 mL), Pearlman's catalyst (55 mg) was added and the reaction mixture was heated under reflux (neutral atmosphere) for 5 h. The reaction mixture was filtered twice through Celite and the Celite was washed with hot ethanol. Evaporation yielded the product (81 mg), which was subsequently purified on a silica gel column (50% EtOAc in hexane to pure EtOAc). Yield: 67.0 mg (82.1%). $H^1$ NMR (300 MHz; ($CDCl_3$): 9.72 (br s, 1H, NH), 7.56 (br s, 1H, H-6), 6.04 (dd, $J_{1',2A'}$ = 6.9 Hz, $J_{1',2'B}$ = 4.5 Hz, 1H, H-1'), 4.45 (dm, $J_{6A',F}$ = $J_{6'B,F}$ = 47.1 Hz, 2H $CH_2F$), 4.02 - 3.94 (m, 2H, H-4' + H-5'A), 3.70 (dd, $J_{gem}$ = 12.9 Hz, $J_{5'B,4'}$ = 3.9 Hz, 1H, H-5'B), 2.76 (m, 1H, H-3'), 2.32 - 2.12 (m, 2H, 2 x H-2'), 1.82 (br s, 3H, $CH_3$).

The following paragraphs A, B, C and D summarize the processes described in the preceding examples.

A) The process for preparing a 3-'deoxy-3'-substituted methyl nucleoside as defined by Formula I

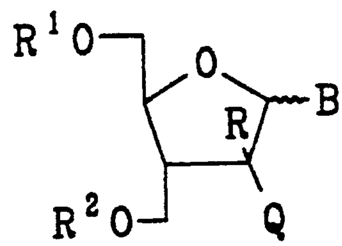

I

wherein B is a nucleoside base as defined above, and R and Q are hydrogen or one of R and Q is hydroxy and the other is hydrogen, which comprises converting 1,2-isopropylidene-3-deoxy-3-$R^2$O-methyl-5-O-$R^1$-α-D-ribofuranose of Formula III, wherein $R^1$ and $R^2$ are independently hydrogen or a hydroxy protecting group

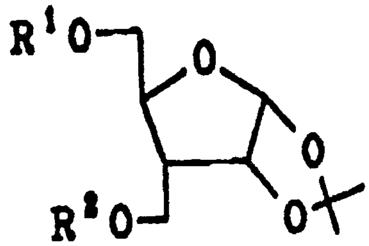

III

into nucleoside base reactive intermediate of Formula IV or Formula XI

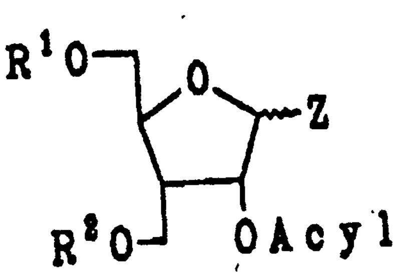

IV

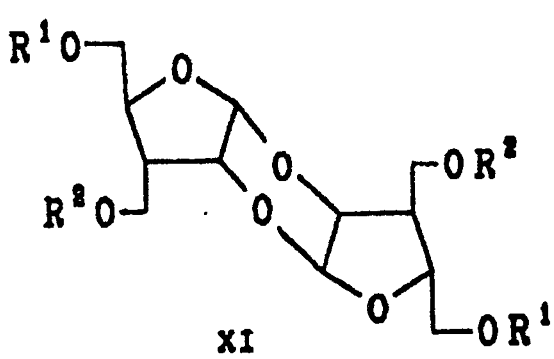

XI

wherein Z is a nucleoside base reactive ester and acyl is an alkanoyl or aroyl group having up to 7 carbon atoms, and thereafter contacting said intermediate of Formula IV or XI with a nucleoside base as defined above with respect to B or an activated derivative thereof under reaction conditions, and thereafter, if desired, removing protective groups, thereby providing a 3′-deoxy-3′-hydroxymethyl nucleoside analog having the formula

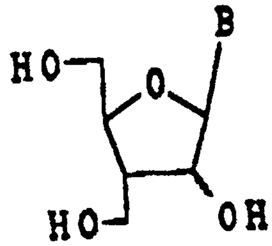

and thereafter, if desired, replacing its 2′-OH group thereof by hydrogen by known means.

B) The process for preparing a 3′-deoxy-3′-substituted methyl nucleoside of Formula XXII

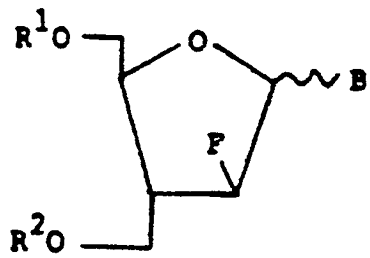

XXII

wherein $R^1$ and $R^2$ are independently hydrogen or hydroxy protecting groups and B is a nucleoside base as defined above, which comprises converting a compound of Formula XXXI into a compound of Formula XXXIV by known means, and hydrogenating the latter under catalytic hydrogenation conditions to provide a compound or Formula XXXV

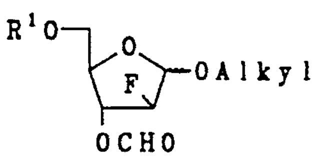

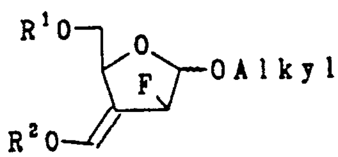

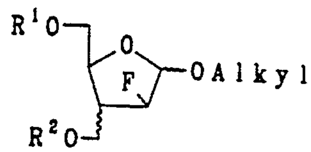

XXXI XXXIV XXXV

wherein $R^1$ and $R^2$ are hydrogen or hydroxy protecting groups and alkyl is an alkyl group having up to 7 carbon atoms and thereafter converting the compound of Formula XXXV into a nucleoside base reactive form having Formula XXI

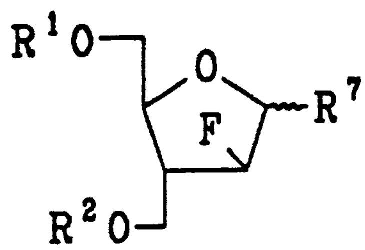

XXI

wherein $R^7$ is bromine or an activated ester group, and thereafter contacting the compound or Formula XXI with a nucleoside base as defined with respect to B or an activated derivative thereof, under reaction conditions to provide said compound of Formula XXII and thereafter, if desired, removing protective groups.

C) The process for preparing a 3′-deoxy-3′-substituted methyl nucleoside of Formula II

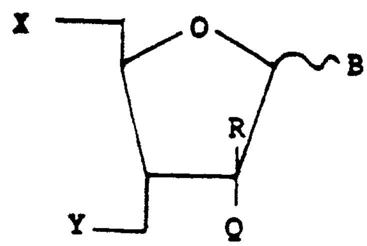

II

wherein X, Y, R, Q, and B are as previously defined, which comprises contacting a compound or Formula V or Formula VII

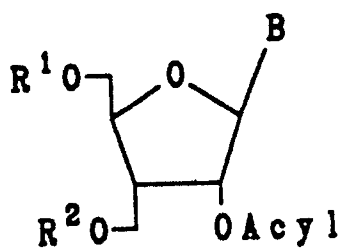

V

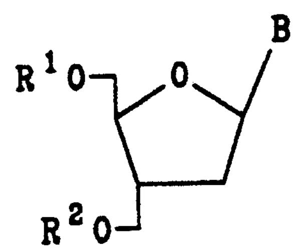

VII

wherein B is a nucleoside base as defined above, acyl is an alkanoyl or aroyl group having up to 7 carbon atoms, and one of $R^1$ and $R^2$ is hydrogen and the other is a hydroxy protecting group with an alkylsulfonyl, arylsulfonyl, or trifluoroalkylsulfonyl acylating agent to provide a sulfonyloxy derivative of the compound of Formula V or VII, wherein one of $R^1$ and $R^2$ in either instance is the alkylsulfonyl, arylsulfonyl, or trifluoroalkylsulfonyl group of said acylating agent, and thereafter contacting said sulfonyloxy derivative with a nucleophilic reagent capable of displacing said sulfonyloxy group with said X or Y group of Formula II, and, if desired, removing said protecting groups.

D) The process for preparing a 3′-deoxy-3′-fluoromethyl nucleoside of Formula XXXIX or XXXX

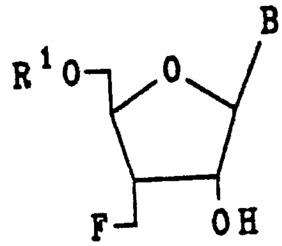

XXXIX

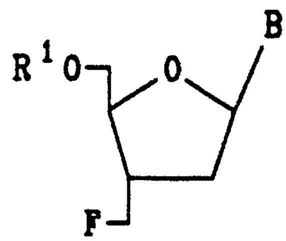

XXXX

wherein B is as previously defined, and R¹ is hydrogen or a hydroxy protecting group which comprises converting 1,2:5,6-di-O-isopropylidene-3-C-hydroxymethyl-α-D-gluco-hexofuranose with diethylaminosulfur trifluoride in tetrahydrofuran solution in the absence of moisture and atmospheric oxygen at room temperature to provide 1,2:5,6-di-O-isopropylidene-3-deoxy-3-C-fluoromethyl-α-D-gluco-hexofuranose, and converting the latter by known means into 1,2-di-O-acetyl-5-O-benzyl-3-deoxy-3-C-fluoromethyl-β-D-ribo-pentofuranose which is then contacted with a nucleoside base as defined above with respect to B in a polar aprotic solvent in the presence of a silylating agent and trifluoromethanesulfonic acid, thereby yielding the 2′-O-acetate of the compound of Formula XXXIX, wherein R¹ is benzyl and thereafter converting said compound to a compound of Formula XXXX, wherein R¹ is benzyl by a known 2′-deoxygenation method, and, if desired, removing protective groups R¹ from the compound of Formula XXXIX or XXXX.

## BIOLOGICAL TESTING

### Testing and Evaluation of Compounds Against Herpes Virus

#### A. Plaque Reduction Assay

Herpes simplex virus (HSV) strains were grown and titered at 37° C in vero cells (African Green Monkey Kidney cells) and used for virus work before the tenth passage.

Cells were grown and maintained in Earle's Minimum Essential Medium (EMEM), Gibco Laboratories, supplemented with 0.75% sodium bicarbonate, 2mM 1-glutamine, Pen-strep. and 5-10% fetal calf serum.

The titer of HSV strains is determined by a plaque titration method (Roizman and Roane, "Virology," 15:75-79, 1961) . Tissue culture 24-well petri dishes are seeded with cells and used for assays with approximately 75% monolayer. Volumes (0.1 mL) of logarithmic dilutions of the virus strain are inoculated onto each of triplicate wells, and absorbed for one hour with intermittent shaking. The inoculum thereafter is removed, and 1 mL of 5-10% EMEM containing 0.3% human immune serum globulin is added. After a 40-hour incubation period at 37° C in a 5% $CO_2$ atmosphere, the overlay medium is removed and the cell sheets stained with Giemsa stain. The number of plaques is counted, the triplicate is averaged, and the number of plaque-forming units per mL is calculated.

The compounds are tested for activity against the herpes simplex strains using a stock solution of each compound freshly prepared. Appropriate dilutions of each compound are made in 10% EMEM before usage. The antiviral efficacy of each compound is determined using the plaque reduction assay described above. Briefly, tissue culture 24-well plates, with approximately 75% cell monolayer are inoculated with approximately 50 plaque forming units of HSV per 0.1 mL, and the virus adsorbed for 1 hour, with intermittent shaking. After removal of the inoculum, 1 mL of 10% EMEM containing two-fold dilutions of the appropriate drug are added in triplicates. Triplicate wells/plate receives no drug and are used as a virus control. After a 48-hour incubation period, at 37° C in a 5% $CO_2$ atmosphere, the overlay medium is removed, the cells stained as described above, and plaques are counted. The counts of triplicate wells are averaged, and the number of plaques in the presence of each drug dilution are calculated.

The antiviral potency of the drug is determined by $ID_{50}$, the drug concentration necessary to reduce the number of plaques by 50% of those in the virus control cultures.

B. Colorimetric Dye-Uptake Assay

For the primary screening, a colorimetric dye-uptake assay (McLaren, C., et al., "Antiviral Research", 3:323, 1983) is used employing rapidly growing vero cells. Briefly, cells, compound and virus dilutions are added onto 96-well tissue culture plates simultaneously using the cells, viruses and medium described above. After 48-hour incubation at 30° C in 5% $CO_2$ atmosphere, the overlay medium is removed and the cell sheets are stained with 0.04% neutral red solution. After 30 minutes, incubation at 30° C, the cell sheets are washed and the stain is eluted with 0.05 M sodium monophosphate in 47% ethanol and the O.D. is determined at 540 nm wavelength.

The 50% inhibitory dose (ID50) for each drug is determined by linear regression analysis.

C. Testing and Evaluating of Compounds Against Human Cytomegalovirus

Human cytomegalovirus (HCMV) strain (AD169) was grown and titered at 37° in human embryonic lung (diploid) cells, MRC-5, and used for the antiviral assay.

The compounds are tested for activity against the HCMV using the procedure for the plaque reduction assay described above.

D. Testing and Evaluating of Compounds Against Murine Retroviruses

The compounds were evaluated for antiviral activity against Murine leukemia virus (MuLV) strains using the UV-XC plaque assay (Rowe, et al, "Virology", 42:1136, 1970).

The MuLV strains were grown in feral mouse cells (SC-1) and used for antiviral tests using the UV-XC plaque assay. Briefly, SC-1 cells are grown as monolayers in 4-well tissue culture plates and inoculated with approximately 50-100 plaque forming units of MuLV in 0.5 mL of 5% EMEM containing 20 ug/mL DEAE/Dextran. After 1 hour adsorption, the inoculum is removed and 5 mL of 5% EMEM containing three-fold dilutions of the appropriate drug are added. Five days later, the cultures are UV-irradiated with an ultraviolet lamp and rat XC sarcoma cells are added to the cultures. Three to four days after UV-irradiation, the cell cultures are stained with Giemsa stain and the plaques are enumerated. Antiviral activity is expressed in terms of the reduction in the mean number of UV-XC plaques counted in the drug treated, virus-infected cultures compared with mean number of plaques counted in untreated, virus-infected control cultures.

E. Testing and Evaluating of Compounds Against HIV

The anti-HIV/LAV activity was measured in cultures of CEM-F cells. The CEM cells were infected with approximately 30 $TCID_{50}$ (50% tissue culture infectious dose) of HIV (LAV strain). The cells were then incubated for 45 minutes at 37° C. The test compounds, in culture medium, were added at various concentrations to the infected cells and then incubated for a further eight days. After eight days, the antiviral activity was evaluated in the culture media supernatant for p-24 gag protein by an enzyme capture assay (ELISA). The antiviral activity was expressed as the dose that inhibits 50% of the virus expression ($ID_{50}$ in $\mu$M) as detected by the assay described.

Some representative antiviral test data are displayed in Table 1.

TABLE I

| Example | HSV-1[1] | HSV-2[1] | MuLV | HIV | HCMV |
|---|---|---|---|---|---|
| 4[3] | 146 | 130 | >32 | - | - |
| 4[2] | 17.1 | >131 | 17.7 | >10 | - |
| 8 | >133 | >133 | >100 | 0.8 | - |
| 12 | >166 | >166 | 83 | >10 | - |
| 16[2] | 1.5 | 37 | 0.06 | >0.1 | 1.7 |
| 16[3] | >50 | >50 | - | 4.7 | - |
| 25 | >165 | >124 | - | >10 | >100 |
| 26 | >166 | >166 | 5.0 | >10 | >100 |
| AZT[4] | - | - | 0.0066 | 1.0 | - |
| ACV[5] | 1 | 1.49 | - | - | 2.3 |

1. As determined by plaque reduction assay or dye uptake assay
2. The beta isomer
3. The alpha isomer
4. 3′-Azido-3′-deoxythymidine
5. Acyclovir

Although the invention has been described in considerable detail with specific reference to certain advantageous embodiments thereof, variations and modifications can be made without departing from the scope of the invention as described in the specification and defined in the appended claims.

## Claims

1. 3′-Substituted methyl nucleosides represented by Formula I

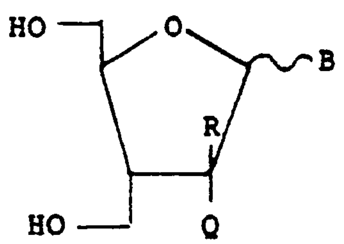

I

wherein:

~ B indicates that B can be either alpha or beta;

B is a member selected from the group consisting of the pyrimidine and purine nucleoside bases connected to the tetrahydrofuran ring through the $N^1$ heterocyclic nitrogen atom of the pyrimidines and the $N^9$ or $N^7$ heterocyclic nitrogen atom of the purines including the naturally occurring nucleoside bases and: 5-fluoro, 5-bromo, 5-iodo, 5-chloro, 5-trifluoromethyl, 5-ethyl, 5-(2-bromovinyl)uracil; triazolecarboxamide; 2-aminopurine; 2,6-diaminopurine; 4-chloropyrimidine; pyrimidine; azapyrimidine; purine; 2,6-dichloropurine; 2-amino-6-chloropurine; and deazapurine;

R and Q are each halogen, or one is hydrogen and the other is halogen, and

the nucleosides of Formula I bearing hydroxy protective groups, and when B comprises a hydroxy or amino group, the protected forms thereof.

2. A 3′-substituted methyl nucleoside as defined by claim 1 having the formula

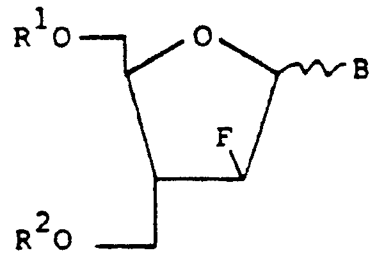

XXII

wherein B is as defined in claim 1, and $R^1$ and $R^2$ are independently selected from hydrogen and a hydroxy protective group.

3. The α anomer of the compound of claim 2.

4. The β anomer of the compound of claim 2.

5. A 3′-substituted methyl nucleoside as defined by claim 1 having the formula

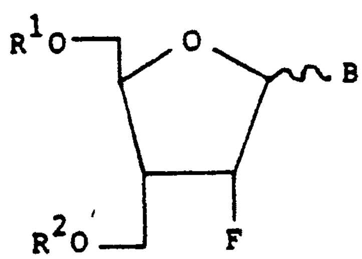

XXVIII

wherein B is as defined in claim 1, and $R^1$ and $R^2$ are independently selected from hydrogen and a hydroxy protective group.

6. The α anomer of the compound of claim 5.

7. The β anomer of the compound of claim 5.

8. A 3′-substituted methyl nucleoside selected from the group consisting of
1-(2,3-dideoxy-3-C-hydroxymethyl-β-D-erythro-pentofuranosyl)thymine,
1-(2,3-dideoxy-3-C-hydroxymethyl-α-D-erythro-pentofuranosyl)thymine,
1,(2,3-dideoxy-3-C-hydroxymethyl-β-D-erythro-pentofuranosyl)cytosine,
1-(2,3-dideoxy-3-C-hydroxymethyl-α-D-erythro-pentofuranosyl)cytosine,
1-(3-deoxy-3-C-hydroxymethyl-β-D-arabinofuranosyl)cytosine,
9-[2,3-dideoxy-3-C-hydroxymethyl-β-D-erythro-pentofuranosyl]adenine,
9-(2,3-dideoxy-3-C-hydroxymethyl-β-D-erythro-pentofuranosyl)hypoxanthine,
2,3-dideoxy-3-azidomethyl-β-D-erythro-furanosyl)thymine,
1-(3-deoxy-3-C-hydroxymethyl-β-D-ribofuranosyl)cytosine,
9-(3-deoxy-3-C-hydroxymethyl-β-D-ribopentofuranosyl)guanine,
9-(2,3-dideoxy-3-C-hydroxymethyl-β-D-ribopentofuranosyl)guanine.

9. 3′-Substituted methyl nucleosides represented by Formula II

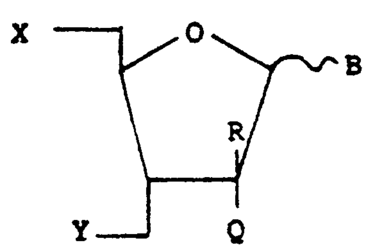

II

wherein:
~ B indicates that B can be either alpha or beta;
B is a member selected from the group consisting of the pyrimidine and purine bases connected to the tetrahydrofuran ring through the $N^1$ heterocyclic nitrogen atom of the pyrimidines and the $N^9$ or $N^7$ heterocyclic nitrogen atom of the purines including the naturally occurring nucleoside bases and: 5-fluoro, 5-bromo, 5-iodo, 5-chloro, 5-trifluoromethyl, 5-ethyl, 5-(2-bromovinyl)uracil; triazolecarboxamide; 2-aminopurine; 2,6-diaminopurine; 4-chloropyrimidine; pyrimidine; azapyrimidine; purine; 2,6-dichloropurine; 2-amino-6-chloropurine; and deazapurine;
R and Q are both hydrogen, or both halogen, or one is hydrogen and the other is hydroxy or halogen, and X and Y can be the same or different and are substituents selected from the group consisting of acylamido, alkoxy, acyloxy or alkylthio, each having 1-6 carbon atoms, halogen, azido, amino, -SH, and aralkoxy, aryloxy or arylthio, each having 6-10 carbon atoms, or one of X or Y is one of said substituents and the other is hydroxy, or protected hydroxy, except when X is O-acyl.

10. A 3′-substituted methyl nucleoside selected from the group consisting of 1-(2,3-dideoxy-3-azidomethyl-β-D-erythro-furanosyl) thymine,
1-(2,3-dideoxy-3-azidomethyl-β-D-erythro-furanosyl)uracil,
1-(2,3-dideoxy-3-C-fluoromethyl-β-D-erythro-pentofuranosyl)thymine.

11. The use of at least one of the compounds of anyone of claims 1 to 10 for preparing a pharmaceutical composition for treating a viral infection.

12. An intermediate useful in the production of a 3′-substituted methyl nucleoside selected from the group consisting of
1,2,5-tri-O-acetyl-3-deoxy-3-[(acetoxy)methyl]-β-D-ribofuranose,
methyl 5-O-benzyl-2,3-dideoxy-3-[(benzyloxy)methyl-β-D-erythro-pentofuranoside,
1-[2,5-di-O-acetyl-3-deoxy-3-[(acetoxy)methyl]-β-D-ribofuranosyl)uracil,
1-[2,5-di-O-acetyl-3-deoxy-3-[(acetoxy)methyl-β-D-ribofuranosyl]cytosine,
1-[2-O-acetyl-5-O-benzyl-3-deoxy-3-[(benzyloxy)methyl)-β-D-ribofuranosyl]uracil,
1-[5-O-benzyl-3-deoxy-3-[(benzyloxy)methyl-β-D-ribofuranosyl]uracil,
1-[5-O-benzyl-2,3-dideoxy-3-[(benzyloxy)methyl]-β-D-erythro-pentofuranosyl]uracil
1-[5-O-benzyl-3-deoxy-3-[(benzyloxy)methyl]-β-D-ribofuranosyl]-4-thiouracil,
1-[5-O-benzyl-3-deoxy-3-[(benzyloxy)methyl-β-D-ribofuranosyl]cytosine,
1-[5-O-benzyl-3-(benzyloxy)methyl-2,3-dideoxy-β-D-erythro-furanosyl)thymine,
1-[5-O-benzyl-2,3-dideoxy-3-(methanesulfonyloxy)-methyl-β-D-erythro-furanosyl)thymine,
1-[5-O-benzyl-2,3-dideoxy-3-[(benzyloxy)methyl]-β-D-erythro-pentofuranosyl]uracil,
5-O-benzyl-3-deoxy-3-[(benzyloxy)methyl]-1,2-anhydro-α-D-ribofuranose,
1-[5-O-benzyl-3-deoxy-3-[(benzyloxy)methyl]-β-D-ribofuranosyl]thymine,
5′-O-benzyl-3′-deoxy-3′-[(benzyloxy)methyl]-2,2′-anhydro-β-D-uridine.
1-[5-O-benzyl-3-deoxy-3-[(benzyloxy)methyl]-β-D-arabinofuranosyl]uracil,
1-[2,5-di-O-acetyl-3-deoxy-3-[(acetoxy)methyl-β-D-arabinofuranosyl]-4-thiouracil,
9-[5-O-benzyl-3-deoxy-3-[(benzyloxy)methyl]-β-D-ribofuranosyl]adenine,
9-[5-O-benzyl-2,3-dideoxy-3-[(benzyloxy)methyl]-β-D-ribofuranosyl]adenine.,
5′-O-benzyl-3′-deoxy-3-[(benzyloxy)methyl]-1,2-O-isopropylidene-α-D-ribofuranose,
1,2-di-O-acetyl-5-O-benzyl-3-deoxy-3-[(benzyloxy)methyl-α- and β-D-ribofuranose,
1-(2-O-acetyl-5-O-benzyl-3-deoxy-3-[(benzoyloxy)methyl-β-D-ribofuranosyl)thymine,
1-[5-O-benzyl-3-deoxy-3-[(benzoyloxy)methyl]-β-ribofuranosyl]thymine,
$N^2$-acetyl-9-(3-deoxy-3-[(benzyloxy)methyl]-β-D-ribo-pentofuranosyl)guanine,
$N^2$-acetyl-9-(2-O-acetyl-3-deoxy-3-C-hydroxymethyl]-β-D-ribo-pentofuranosyl)guanine,
9-(5-O-benzyl-3-deoxy-3-[(benzyloxy)methyl]-β-D-ribo-pentofuranosyl)guanine,
1-(2-O-acetyl-5-O-benzyl-3-deoxy-3-[(benzoyloxy)methyl]-β-D-ribofuranosyl)uracil,
1-(5-O-benzyl-3-deoxy-3-[(benzoyloxy)methyl]-2-hydroxy-β-D-ribofuranosyl)uracil,
1-(5-O-benzyl-3[(benzoyloxy)methyl]-2,3-dideoxy-β-D-erythro-furanosyl)uracil,
1-(5-O-benzyl-2,3-dideoxy-3[(methanesulfonyl)methyl]-β-D-erythro-furanosyl)uracil,
1(2,3-dideoxy-3-[(methanesulfonyl)methyl]-β-D-erythro-furanosyl)uracil,
1,2-Di-O-acetyl-5-O-benzyl-3-deoxy-3-C-fluoromethyl-β-D-ribo-pentofuranose,
1-(2-O-acetyl-5-O-benzyl-3-deoxy-3-C-fluoromethyl-ribo-pentofuranosyl)thymine,
1-(5-O-benzyl-3-deoxy-3-C-fluoromethyl-β-D-ribo-pentofuranosyl)thymine,
1-(5-O-benzyl-2,3-dideoxy-3-C-fluoromethyl-β-D-erythro-pentofuranosyl)thymine.

13. The process for preparing a 3-′deoxy-3′-substituted methyl nucleoside as defined by Formula I

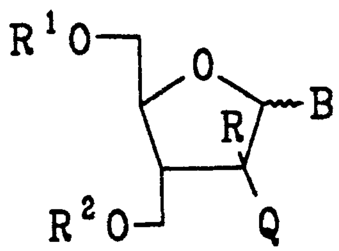

I

wherein B is a nucleoside base as defined above, and R and Q are hydrogen or one of R and Q is hydroxy and the other is hydrogen, which comprises converting 1,2-isopropylidene-3-deoxy-3-$R^2$O-methyl-5-O-$R^1$-α-D-ribofuranose of Formula III, wherein $R^1$ and $R^2$ are independently hydrogen or a hydroxy protecting group

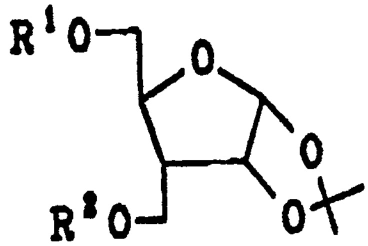

III

into a nucleoside base reactive intermediate of Formula IV or Formula XI

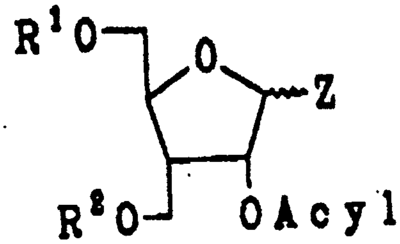

IV

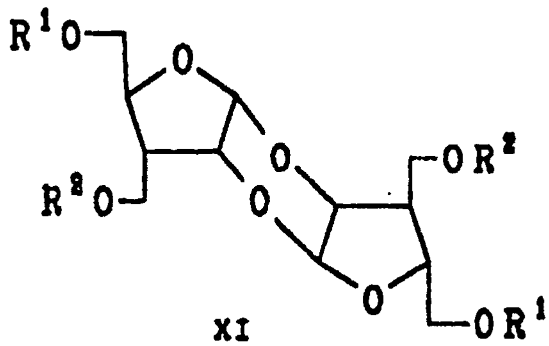

XI

wherein Z is a nucleoside base reactive ester and acyl is an alkanoyl or aroyl group having up to 7 carbon atoms, and thereafter contacting said intermediates of Formula IV or XI with a nucleoside base as defined above with respect to B or an activated derivative thereof under reaction conditions, and thereafter, if desired, removing protective groups, thereby providing a 3′-deoxy-3′-hydroxymethyl nucleoside analog having the formula

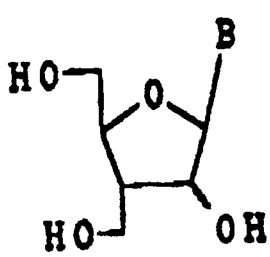

and thereafter, if desired, replacing its 2′-OH group thereof by hydrogen by known means.

14. The process for preparing a 3′-deoxy-3′-substituted methyl nucleoside of Formula XXII

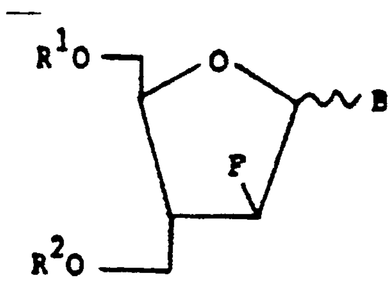

XXII

wherein $R^1$ and $R^2$ are independently hydrogen or hydroxy protecting groups and B is a nucleoside base as defined above, which comprises converting a compound of Formula XXXI into a compound of Formula XXXIV by known means, and hydrogenating the latter under catalytic hydrogenation conditions to provide a compound of Formula XXXV

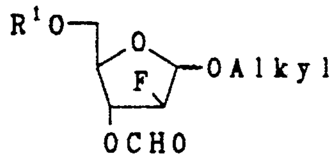

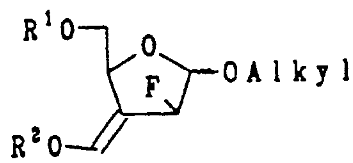

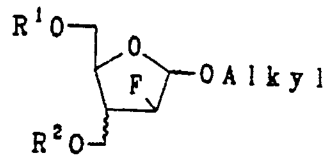

XXXI XXXIV XXXV

wherein $R^1$ and $R^2$ are hydrogen or hydroxy protecting groups and alkyl is an alkyl group having up to 7 carbon atoms and thereafter converting the compound of Formula XXXV into a nucleoside base reactive form having Formula XXI

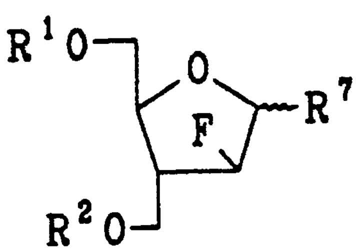

XXI

wherein $R^7$ is bromine or an activated ester group, and thereafter contacting the compound of Formula XXI with a nucleoside base as defined with respect to B or an activated derivative thereof, under reaction conditions to provide said compound of Formula XXII and thereafter, if desired, removing protective groups.

15. The process for preparing a 3′-deoxy-3′-substituted methyl nucleoside of Formula II

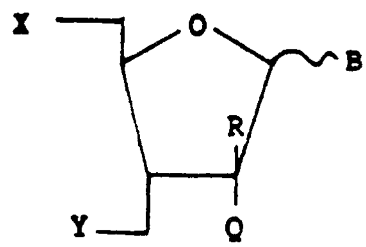

II

wherein X, Y, R, Q, and B are as previously defined, which comprises contacting a compound of Formula V or Formula VII

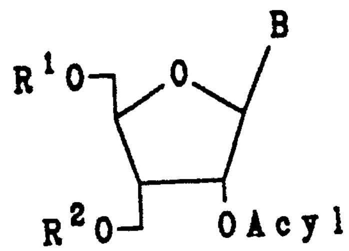

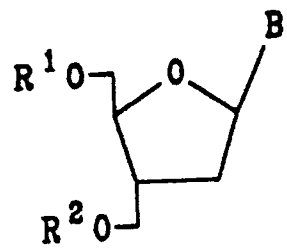

V VII

wherein B is a nucleoside base as defined above, acyl is an alkanoyl or aroyl group having up to 7 carbon atoms, and one of $R^1$ and $R^2$ is hydrogen and the other is a hydroxy protecting group with an alkylsulfonyl, arylsulfonyl, or trifluoroalkylsulfonyl acylating agent to provide a sulfonyloxy derivative of the compound of Formula V or VII, wherein one of $R^1$ and $R^2$ in either instance is the alkylsulfonyl, arylsulfonyl, or trifluoroalkylsulfonyl group of said acylating agent, and thereafter contacting said sulfonyloxy derivative with a nucleophilic reagent capable of displacing said sulfonyloxy group with said X or Y group of Formula II, and, if desired, removing said protecting groups.

16. The process for preparing a 3′-deoxy-3′-fluoromethyl nucleoside of Formula XXXIX or XXXX

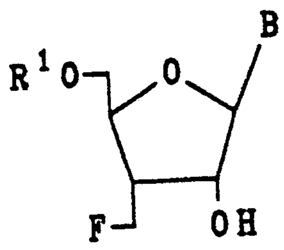

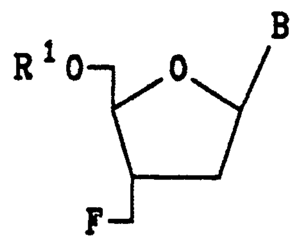

XXXIX XXXX

wherein B is as previously defined, and $R^1$ is hydrogen or a hydroxy protecting group which comprises converting 1,2:5,6-di-O-isopropylidene-3-C-hydroxymethyl-α-D-gluco-hexofuranose with diethylaminosulfur trifluoride in tetrahydrofuran solution in the absence of moisture and atmospheric oxygen at room temperature to provide 1,2:5,6-di-O-isopropylidene-3-deoxy-3-C-fluoromethyl-α-D-gluco-hexofuranose, and converting the latter by known means into 1,2-di-O-acetyl-5-O-benzyl-3-deoxy-3-C-fluoromethyl-β-D-ribo-pentofuranose which is then contacted with a nucleoside base as defined above with respect to B in a polar aprotic solvent in the presence of a silylating agent and trifluoromethanesulfonic acid, thereby yielding the 2′-O-acetate of the compound of Formula XXXIX, wherein $R^1$ is benzyl and thereafter converting said compound to a compound of Formula XXXX, wherein $R^1$ is benzyl by a known 2′-deoxygenation method, and, if desired, removing protective groups $R^1$ from the compound of Formula XXXIX or XXXX.

17. A pharmaceutical composition comprising at least one compound of anyone of claims 1 to 10, optionally together with pharmaceutically acceptable carriers and/or adjuvants.

18. A process for preparing the pharmaceutical composition of claim 17 which comprises optionally incorporating at least one compound of anyone of claims 1 to 10 into a pharmaceutically acceptable carrier and/or adjuvant and providing said compound in a form suitable for administration.